# EUROPEAN PATENT APPLICATION

(11) **EP 1 378 751 A2**
(43) Date of publication of application: **07.01.2004**
(21) Application number: 03014486.9
(22) Date of filing: 02.07.2003
(51) Int. Cl.: G01N 33/53, G01N 33/543

(54) **Method of measuring drug-metabolizing enzyme activity, method of evaluating inhibition of drug-metabolizing enzyme activity, and composition for these methods**

(30) Priority: 02.07.2002 JP 2002193795; 02.07.2002 JP 2002193796; 06.08.2002 JP 2002229065; 06.08.2002 JP 2002229066; 06.08.2002 JP 2002229067
(71) Applicant: Toyo Boseki Kabushiki Kaisha, Osaka-shi, Osaka-fu (JP)
(72) Inventor: Matsui, Kazuhiro, Tsuruga Inst. of Biotechnology, Tsuruga-shi, Fukui-ken (JP); Ishibashi, Takuya, Tsuruga Inst. of Biotechnology, Tsuruga-shi, Fukui-ken (JP); Oka, Masanori, Tsuruga Inst. of Biotechnology, Tsuruga-shi, Fukui-ken (JP)
(74) Representative: VOSSIUS & PARTNER

(57) **Abstract**

The present invention provides a method of measuring drug-metabolizing enzyme activity, wherein a drug-metabolizing enzyme is first applied to a substrate (particularly an endogenous unmodified substrate), and measurement is performed preferably within three hours by immunochemical assay of the resulting product.

## Description

### BACKGROUND OF THE INVENTION

### 1. Field of the Invention

The present invention relates to a method of measuring the degree to which a certain substance or substances are affected by other substance or substances which may have an effect on an intended action of said certain substance or substances. Moreover, it relates to measurement of the activity of drug-metabolizing enzymes, particularly those in the cytochrome P450 enzyme family and hydroxysteroid dehydrogenase, and to a method of evaluating the inhibitory effects of chemical substances and the like on drug-metabolizing enzymes. The present invention also relates to a composition which is used in constructing measuring systems in accordance with these methods.

The present invention also relates to a method of evaluating differences between individuals and between specific groups of humans in the activity of metabolizing enzymes with respect to drugs, and to a method of evaluating differences between individuals and between specific groups of humans in inhibition of metabolizing enzyme activity with respect to drugs. Of the drug-metabolizing enzymes, the present invention deals particularly with mutants and single nucleotide polymorphisms. The present invention also relates to a composition used in the construction of measuring systems in accordance with these methods.

### 2. Description of the Related Art

Of the drug-metabolizing enzymes, cytochrome P450 is an enzyme which regulates levels of hormones involved in reproduction, sexual differentiation, maturation, growth, metabolism, maintenance and the like, and it is also involved in detoxification in the body since it metabolizes a variety of drugs and toxins. The literature on cytochrome P450 is vast, with a wealth of data regarding its effect on a variety of drugs; recent years have seen new findings on single nucleotide polymorphisms (SNPs) and subgroups of cytochrome P450, and research continues to progress worldwide. As a result, more is being discovered about the mechanisms of expression, structures, functions, and evolutionary mechanisms of cytochrome P450 of various origins, as well as differences in SNPs among human races. The same is true of other drug-metabolizing enzymes such as hydroxysteroid dehydrogenase, alcohol dehydrogenase, aldehyde dehydrogenase, monoamine oxidase, xanthine oxidase and other oxidoreductases and conjugation enzymes such as UDP-glucuronic acid transferase, UDP-sulfonic acid transferase, sugar transferase, glutathione reductase, and as research keeps up with the accelerating pace of drug discovery, more is being learned about the metabolic pathways, kinetics, side-effect mechanisms, subgroups and polymorphisms of drugs.

In recent years there have also been cases in which the effects not only of drugs but also of widely-used chemical substances, unintentional by-products and chemical contaminants have been mediated by P450 enzymes. In terms of effects on the reproductive system, cases are known in which genetically female organisms have become to have a male reproductive organ (cytochrome P450 aromatase (CYP19) inhibition). This abnomal change is generally called "imposex". Horiguchi et al *(Mar. Environ. Res. Vol. 50,* pp 223-229 (2000)) have reported on such cases in shellfish. Since the end of the last century, the effects of man-made chemicals (so-called endocrine disrupting chemicals) on natural gender and reproduction have represented an urgent problem for the human race. In some cases, P450 aromatase is also closely associated with cancers such as breast and uterine cancers. P450 aromatase is expressed in the course of estrogen-dependent cancers, and recently Kitawaki et al have reported that aromatase is expressed in conjunction with endometriosis, uterine gland myoma and hysteromyoma (Kitawaki, J. et al., *Biol. Reprod.* Vol. 57, 514-519 (1997)). They confirmed by immune staining using aromatase-specific antibodies that aromatase is not found in the endometria of healthy women. Measurement of P450 aromatase activity is also considered useful in determining hormone sensitivity and developing treatment plans in the treatment of benign mammary disorders, breast cancer and the like. The presence of aromatase has also been confirmed in the placenta, ovaries, Sertoli cells, Leydig's cells, fat tissue, muscle and hair and even in the brain, and it has been suggested that it may be involved in sexual behavior and the sexual differentiation of the brain.

Many unintended by-products and chemical contaminants such as benzopyrenes in tobacco smoke and polycyclic aromatic compounds in various kinds of exhaust gas are metabolized by P450 enzymes, and in some cases may be converted to even more toxic chemical compounds in the body. Some metabolites can become tumor promoters.

Some human cytochrome P450s exhibit differences (single nucleotide polymorphisms or SNPs) in their nucleotide and amino acid sequences between individuals or between races, and the response to drugs may also be different. Databases of those SNPs which have been identified are available for example on the web site of the U.S. National Institutes of Health. Most SNPs are known only as base sequences or amino acid sequences, but better data on reactivity for individual drugs should be available in the future. Tailor-made therapies and drug regimens should also be possible based on new information about individual P450 gene groups. Inhibition of cytochrome P450 activity is important because it raises the possibility of drug side-effects. Consequently, it is extremely important that inhibitory effects be confirmed when selecting candidate compounds in the process of drug development. In some cases, serious interactions are reported after a drug has been developed and is on the market. In order to avoid the social, human and economic consequences of side-effects, the interactions of candidate compounds need to be identified during the early stages of drug development, which also makes the entire development process speedier and more efficient. For these reasons, drug manufacturers are putting great effort into screening for inhibition of drug-metabolizing enzyme activity and particularly cytochrome P450 activity as part of essential preclinical test for drug interactions.

Polymorphisms including SNPs, gene deletions and gene duplications are known for human cytochrome P450 CYP2D6, CYP2C9, CYP2C19 and the like, in terms of differences in both genotype and phenotype (Rodrigues, A.D. et al, *Curr. Drug Metab. Vol. 3,* pp 289-309 (2002)). The reactivity of aromatase is also known to depend on a difference (isoleucine or methionine) in amino acid 133 of the substrate recognition site (Conley, A. et al, *Mol. Endocrinol. Vol. 16,* pp 1456-1468 (2002)).

Looking at polymorphic mutants of P450 1B1, 8 substitutions of amino acid positions 48, 119 and 432 are expressed in *E. coli,* and differences in the metabolic reaction for 17β-estradiol or benzopyrene have been studied as reported by Shimada et al (Shimada, T. et al, *Xenobiotica Vol. 31,* pp 163-176 (2001)). These are complex mutations rather than single nucleotide polymorphisms, and an evaluation of the reactivity of such subgroups to various drugs is pharmacologically important.

Cytochrome P450 enzymes are fundamental enzymes which are found in a wide variety of organisms including mammals, birds, reptiles, amphibians, fish, shellfish and other invertebrates, plants and molds and other eukaryotic microorganisms in addition to humans. This means that synthetic chemicals which inhibit or promote P450 enzymes are likely to affect a wide range, not just a specific organism. It is necessary not only to screen drugs but also to re-evaluate the effects of chemical substances already in widespread use, but current testing methods have a variety of problems and new and more efficient methods are needed.

For example, CYP51 is a widely-occurring cytochrome P450 enzyme found in fungi and other microorganisms, plants, mammals and the like, but the sequence of CYP51 differs from species to species, and so does its reactivity to a variety of drugs. By exploiting these species differences, it would be possible to develop and set doses for fungicides that would be effective against fungus but have no effect on humans. Evaluating systems for various CYP51 inhibitors would be useful in the development of new fungicides.

Inhibition of cytochrome P450 activity is also significant because of the possibility of drug side-effects. Consequently, confirming the presence or absence of inhibitory effects is extremely significant when selecting candidate compounds in the process of drug development. In order to avoid the social, human and economic consequences of side-effects, the interactions between candidate compounds and P450s need to be identified during the early stages of drug development, which also makes the entire development process speedier and more efficient. For these reasons, drug manufacturers are putting great effort into screening for inhibition of drug-metabolizing enzyme activity and cytochrome P450 activity in particular as part of essential preclinical test for drug interactions.

Cytochrome P450 is being produced by recombination and other techniques using insect cells, mammalian cells, yeasts, *E. coli* and the like for purposes of testing inhibition (Sigle, R.O. et al, *B. B. R. C. Vol. 201,* pp 201-700 (1994) or Crespi et al, *Adv. Pharmacol. Vol. 43,* pp 171-88 (1997), etc.). Some of these are already on the market as commercial products (E.P. Guengerich, *Nature Reviews Drug Discovery Vol. 1,* pp 359-366 (2002)). Drug metabolizing enzymes other than cytochrome P450 have also been extracted as active forms and they are produced by recombinants which hold their genes from sources such as humans, microorganisms and plants and are available in usable form.

Methods of measuring the cytochrome P450 enzyme aromatase (CYP19) include methods using cells, such as Schenkel et al's method using animal cells (Schenkel et al, *J. Steroid Biochem. Vol. 33,* pp 125-131 (1989)), a screening method using enzymes expressed by yeast cells (Ponpon, D. et al, *Molecular Endocrinology Vol. 3,* pp 1477-1487 (1989)), and a method using mammalian cells made to express aromatase by recombinant techniques (WO91/05794). Such tests can be performed in vitro, but since whole cells are used, the direct reactivity of the cytochrome P450 protein with the tested chemical substance is not evaluated. In terms of measuring aromatase activity, methods of detecting the activity of the aromatase protein itself include the tritium water free assay method using a substrate with a radio isotope label (Bellino, F.L. et al, *J. Clin. Endocrinol. Metab. Vol. 44,* pp 699 (1977)), Crespi et al's fluorescence method of using a fluorescent substrate after metabolization with cytochrome P450 (Crespi, C.L. et al, *Anal. Biochem. Vol. 248,* pp 188-190 (1997)), and Stresser et al's fluorescence method in which a fluorescent substrate is used after metabolization with aromatase (Stresser, D.M. et al, *Anal. Biochem. Vol. 284*, pp 427-430 (2000)). In addition, there are also methods such as that of Taniguchi et al (Taniguchi, H. et al, *Anal. Biochem. Vol. 181,* pp 167-171 (1989)) of isolating, detecting and assaying the product of the enzyme reaction by high perfomance liquid chromatography (HPLC).

Drug-metabolizing enzymes other than aromatase, such as other cytochrome P450 enzymes, 17β-hydroxysteroid dehydrogenase, monoamine oxidase and UDP-glucuronic acid transferase, are also measured and their inhibitory effects evaluated in ways similar to those given above for aromatase. 17β-hydroxysteroid dehydrogenase, monoamine oxidase and UDP-glucuronic acid transferase are also pharmaceutically important, and more data on their measurement and inhibition by various drugs would be useful.

As shown above, evaluation of differences between individuals (when all individual humans share the metabolizing enzyme) and differences between specific groups (when all individuals in the specific groups share the metabolizing enzyme) in the effects (metabolizing activity) of a metabolizing enzyme on various drugs, and evaluation of differences between individuals or specific groups in the ability of various drugs to inhibit the activity of the metabolizing enzyme is an extremely important research method not only for purposes of efficient screening in the development of tailor-made therapies, drug regimens and pharmaceuticals, but also for re-evaluating the effect of chemicals which are already in widespread use.

In drug development in particular, if the metabolizing enzyme of interest is a single nucleotide polymorphism (one that occurs in at least 1% of the population) of the basic nucleotide sequence of the drug-metabolizing enzyme, or a mutation of the basic nucleotide sequence, it becomes even more important because it can be analyzed with the help of existing SNP data.

Of course, vast numbers of measurements are needed in order to obtain such data, but prior methods of measuring drug-metabolizing enzyme activity have various problems as discussed above and are not suitable for practical, high throughput use.

When using cells such as mammalian cells, recombinant yeast cells or recombinant insect cells, the greatest obstacle in the measurement of drug-metabolizing enzyme activity is the potential effects of other metabolizing enzymes in the cells. A variety of enzymes other than cytochrome P450 are present in type cells, and it is likely that the chemical being tested will be metabolized by such enzymes. Moreover, because the cell membrane permeability of many chemicals is different in animal, yeast and *E. coli* cells, results obtained from such cells will be different, so using cells introduces a bias into the test results.

In the case of methods using tritium labeling or other isotopes, test feasibility is limited because the use of radioactive compounds necessitates special isolation areas and equipment, and care must be taken in disposing of assay waste because it includes radioactive material.

The methods of Crespi et al and Stresser et al employ no radioactive compounds and present few problems when used to measure cytochrome P450 activity, but in the screening of cytochrome P450 inhibitors, many of the chemicals being tested are fluorescent in their own right and self-fluorescence interferes with measurement when creating an dose inhibitory curve for a chemical substance. This method is limited for screening purposes because so many chemicals are fluorescent.

The liquid chromatography method of Taniguchi et al employs no radioactive compounds, and in screening inhibitors, because isolation and analysis are performed by liquid chromatography, there should in theory be no effect from self-fluorescence of the tested substance. However, the need for deproteinization and isolation steps before chromatographic analysis makes this method complicated, and it is incapable of analyzing multiple samples at the same time.

### SUMMARY OF THE INVENTION

After painstaking research into finding a method of measuring drug-metabolizing enzyme (particularly cytochrome P450) activity without using radioactive compounds, without any influence from the effects of self-fluorescence of the tested chemical substance and without the need for complex operations, as well as a method of screening inhibitors of drug-metabolizing enzyme (particularly cytochrome P450) activity, the inventors realized that the problems could be solved by applying drug-metabolizing enzyme to a substrate such as an unmodified endogenous substrate, and using an immunochemical method to measure the product resulting from conversion by the drug-metabolizing enzyme, and perfected the invention of this application.

Namely, the present invention consists of the following.
[1] A measurement method which is a method of evaluating the degree to which a certain substance or substances are affected by other substance or substances which may have an effect on intended action of said certain substance or substances wherein said other substance or substances are first applied, and then the intended action of said certain substance or substances is determined by assaying the resulting product by an immunochemical method.
[2] A measurement method which is a method of evaluating the effect of a third substance or substances on the effect that other substance or substances have on the intended action of a certain substance or substances, comprising the steps of:
   applying said other substance or substances to said certain substance or substances in the presence of the third substance or substances; and
   measuring changes in the amount of the resulting product by an immunochemical method to determine the intended action of said certain substance or substances.
[3] A method of measuring a drug-metabolizing enzyme activity the method comprising the steps of:
   applying the drug-metabolizing enzyme to a substrate or substrates (particularly an endogenous unmodified substrate or substrates); and
   assaying the resulting product by an immunochemical method, preferably within 3 hours.
[4] A method of measuring drug-metabolizing enzyme activity comprising the steps of:
   applying the drug-metabolizing enzyme to a substrate or substrates (particularly an endogenous unmodified substrate or substrates) in the presence of a chemical substance or substrates to be evaluated; and
   determining changes in the amount of the resulting product by an immunochemical method, with preferably at least 10 samples, more preferably at least 28 samples, or further more preferably at least 96 samples measured within 3 hours, and the effect of the chemical substance of interest is evaluated, to evaluate the effect of the chemical substance to be evaluated.

[23] A reagent composition for measuring the degree to which a certain substance or substances are affected by other substance or substances which may have an effect on an intended action of said certain substance or substances, the reagent composition comprising (1)-(3):
(1) other substance or substances which may have an effect on the intended action of said certain substance or substances,
(2) at least one antibody which specifically recognizes the product that results when a certain substance or substances are affected by other substance or substances which may have an effect on an intended action of said certain substance or substances,
(3) at least one reagent which allows measurement using the antibody described in (2) according to one of the methods consisting of enzyme immunoassay, surface plasmon resonance, micro-differential thermal measurement or quartz resonance.

[24] A kit for measuring the degree to which a certain substance or substances are affected by other substance or substances which may have an effect on an intended action of said certain substance or substances, the component parts of which are selected as needed from among at least one plate immobilizing a specific antibody or antibodies (or products cross-linked with protein), gold thin-film chips immobilizing specific antibodies (or products cross-linked with proteins), quartz resonators immobilizing specific antibodies (or product cross-linked proteins), at least one enzyme reaction buffer, at least on, at least one sample diluent, at least one standard substance, at least one tracer enzyme substrate liquid for assaying the tracer enzyme, and the like.

[25] The kit according to [24], wherein the other substance is aromatase.

[26] The method of [3] or [4] for evaluating non-steroid chemical substances.

[27] The method of [3] or [4], wherein the substrate concentration during the drug-metabolizing enzyme reaction is 0.05 nM to 500 µM.

[28] A method of evaluating differences between individuals or between groups of humans in metabolic enzyme activity with respect to a drug, comprising the steps of:
applying a drug-metabolizing enzyme to the drug; and
assaying the resulting product by an immunochemical method to determine an activity of the drug-metabolizing enzyme.

[29] A method of evaluating differences between individuals or between groups of humans in inhibition of drug-metabolizing enzyme activity with respect to a drug, comprising the steps of:
applying the drug-metabolizing enzyme to a substrate or substrates in the presence of a chemical substance or substances to be evaluated; and
measuring changes in the amount of the resulting product by an immunochemical method to assay the activity of the drug-metabolizing enzyme.

[30] The measurement method of [28] or [29], wherein a drug-metabolizing enzyme present in an individual or shared by a specific group of humans is a single nucleotide polymorphism (one occurring in 1% or more of the total population) of the basic nucleotide sequence of the drug-metabolizing enzyme.

[31] The measurement method of [28] or [29], wherein a drug-metabolizing enzyme present in an individual or shared by a specific group of humans has a mutation in its basic nucleotide sequence of the drug-metabolizing enzyme.

[32] A reagent composition for evaluating differences between individuals or between specific groups of humans in metabolic enzyme activity with respect to a drug, the composition comprising (1)-(3) below:
(1) at least one drug-metabolizing enzyme;
(2) at least one antibody which specifically recognizes the product formed by the action of the drug-metabolizing enzyme from a certain substance or substances; and
(3) at least one reagent which allows measurement of the product using the antibody described in (2), using one of the methods consisting of enzyme immunoassay, surface plasmon resonance, micro-differential thermal measurement and quartz resonance.

[33] A reagent composition for evaluating differences between individuals or between specific groups of humans in the inhibition of the activity of a metabolizing enzyme with respect to a drug, the composition comprising (1)-(3) below:
(1) at least one drug-metabolizing enzyme;
(2) at least one antibody which specifically recognizes the product formed by the action of the drug-metabolizing enzyme from a certain substance or substances; and
(3) at least one reagent which allows measurement using the antibody described in (2), using one of the methods consisting of enzyme immunoassay, surface plasmon resonance, micro-differential thermal measurement, and quartz resonance.

[34] A kit for evaluating differences between individuals or between specific groups of humans in metabolizing enzyme activity with respect to a drug, the component parts of which are selected as needed from among at least one plate immobilizing a specific antibodies (or products cross-linked with protein), gold thin-film chips immobilizing specific antibodies (or products cross-linked with proteins), quartz resonators fixed with specific antibodies (or product cross-linked proteins), specific antibody or antibodies for a product or products by metabolic enzymes or at least one gold thin-film chip immobilizing a specific antibody or antibodies for a product or products, or at least one quartz resonator chip fixed with a specific antibody or antibodies, at least one enzyme reaction buffer, at least on, at least one sample diluent, at least one standard substance, at least one tracer enzyme substrate liquid for assaying the tracer enzyme, and the like.

[35] A kit for evaluating differences between individuals or between specific groups of humans in inhibition of metabolizing enzyme activity with respect to a drug, the component parts of which are selected as needed from among at least one plate immobilizing specific antibodies (or products cross-linked with protein), gold thin-film chips immobilizing specific antibodies (or products cross-linked with proteins), quartz resonators immobilizing specific antibodies (or product cross-linked proteins), at least one enzyme reaction buffer, at least on, at least one sample diluent, at least one standard substance, at least one tracer enzyme substrate liquid for assaying the tracer enzyme, and the like.

### BRIEF DESCRIPTION OF THE DRAWINGS

Figure 1 shows a model of the measurement principles of a competitive enzyme immunoassay using immobilized antibody and cross-linked product-tracer enzyme, included in the present invention.
Figure 2 shows a model of the measurement principles of a competitive (cross-linked product-protein complex) enzyme immunoassay included in the present invention.
Figure 3 shows a model of a dose-response curve obtained when screening inhibitors in the present invention.
Figure 4 shows a model of the measurement principles of the surface plasmon resonance method included in the present invention.
Figure 5 shows the relationship between the concentration sequence and resulting absorbance spectrum for human cytochrome P450 CYP19 in Example 1A.
Figure 6 shows the relationship between various concentrations of alpha naphthoflavone solution and absorbance at 450 nm in Example 2A.
Figure 7 shows the relationship between various concentrations of aminoglutethimide solution and absorbance at 450 nm in Example 3A.
Figure 8 shows the relationship between various concentrations of genistein solution and absorbance at 450 nm in Example 4A.
Figure 9 shows the relationship between a concentration sequence of human cytochrome P450 CYP2C19 and the resulting absorbance in Example 5A.
Figure 10 shows the relationships between various concentrations of ketoconazole solution and absorbance at 450 nm in Example 6A.
Figure 11 shows the relationship between various concentrations of ticlopidine solution and absorbance at 450 nm in Example 7A.
Figure 12 shows a model of the measurement principles for the crystal resonance method included in the present invention.
Figure 13 shows the relationship between a concentration sequence of pig cytochrome P450 CYP19 (133I) and the resulting absorbance in Example 1B.
Figure 14 shows the relationship between a concentration sequence of pig cytochrome P450 CYP19 (133M) and the resulting absorbance in Example 1B.
Figure 15 shows the relationship between alpha naphthoflavone solution concentration and absorbance at 450 nm in Example 2B (CYP19 (133I)).
Figure 16 shows the relationship between alpha naphthoflavone solution concentration and absorbance at 450 nm in Example 2B (CYP19 (133M)).
Figure 17 shows the relationship between a concentration sequence of human cytochrome P450 CYP2C9 (359I1e) and the resulting absorbance in Example 3B;
Figure 18 shows the relationship between a concentration sequence of human cytochrome P450 CYP2C9 (359Leu) and the resulting absorbance in Example 3B;
Figure 19 shows the relationship between various concentrations of sulfaphenazole solution and absorbance at 450 nm in Example 4B (CYP2C9 (359Ile)); and
Figure 20 shows the relationship between various concentrations of sulfaphenazole solution and absorbance at 450 nm in Example 4B (CYP2C9 (359Leu)).

### DETAILED DESCRIPTION OF THE INVENTION

Sources for the drug-metabolizing enzyme used in screening for inhibition of drug-metabolizing enzyme in the present invention may be derived from humans, rats, mice or other mammals, chickens or other birds, crocodiles or other reptiles, frogs or other amphibians, trout, killifish or other fish, shellfish, mollusks or other invertebrates, plants, or yeasts, molds or other eukaryotic microorganisms. The drug-metabolizing enzyme which is measured in the present invention may be harvested for example from humans, rats, mice or other mammals, chickens or other birds, crocodiles or other reptiles, frogs or other amphibians, trout, killifish or other fish, shellfish or other invertebrates, plants, or molds, yeasts and other eukaryotic microorganisms.

The drug-metabolizing enzymes covered by the present invention include cytochrome P450, hydroxysteroid dehydrogenase, alcohol dehydrogenase, aldehyde dehydrogenase, monoamine oxidase, xanthine oxidase and other oxidoreductases and UDP-glucuronic acid transferase, UDP-sulfonic acid transferase, sugar transferase, glutathione reductase and other conjugation enzymes as well as hydrolytic enzymes.

These drug-metabolizing enzymes are ones whose metabolic mechanisms in the body are relatively well known at the time of this application, and which are therefore suitable subjects for measurement.

In one preferred embodiment of the present invention, the gist of the technical concept of this application is to use as the "substrate" an unmodified endogenous substrate rather than one which has been modified by isotopes, fluorescent groups or the like, and this does not preclude the future measurement of other metabolizing enzymes about which more will be known in the future as research continues to progress. In particular, HMG-CoA reductase, squalene synthase and other fat metabolizing enzymes, proteases which are critical to the life cycles of microorganisms and viruses such as HIV, HCV and HEV, and metabolizing enzymes such as glutamine synthetase which are only expressed strongly in cancer cells are anticipated, but these do not limit the present invention.

In another preferred embodiment of the present invention, the gist of the technical concept of this application is that by using as the "substrate" an unmodified endogenous substrate rather than one which has been modified by isotopes, fluorescent groups or the like, differences between individuals or between specific groups of humans in metabolizing enzyme activity with respect to a drug can be evaluated, or else differences between individuals or between specific groups of humans in inhibition of the activity of a metabolizing enzyme with respect to a drug can be evaluated, and this does not preclude the future measurement of activity of other drug-metabolizing enzymes about which more will be known in the future as research continues to progress. In particular, it is anticipated that the expression amount and activity of polymorphisms of enzymes such as human P450 1A, 2C enzyme groups and the like which play a primary role in pharmacokinetics, and which are currently subjects of research, will soon become subjects of measurement. It is also anticipated that drug reactivity will be better understood in the case of inborn metabolic abnormalities caused by polymorphisms of specific drug metabolizing enzymes. The study of such polymorphisms should continue to advance not only in the field of drug-metabolizing enzymes but also with respect to all metabolic enzymes involved in fat metabolism and tumor cells.

As in the case of sources of drug-metabolizing enzymes as mentioned above, and as in the case of drug metabolizing enzyme morphology and methods of measuring the products of enzyme reactions as well as the types of reagents used in measurement as discussed below, this is not limited by the state of technology at the time of this application. However, the gist of the technical concept of this application is to use an unmodified endogenous substrate, and this does not in any way preclude the possibility that the selection of other component conditions (including methods of measuring the product of an enzyme reaction, such as specific measurement conditions for the immunochemical method of measuring the product) could favorably influence the effects of the invention of this application.

The types of P450 covered by the present invention include in particular cytochrome P450 consisting of one or a mixture of two or more enzyme proteins selected from the enzyme proteins corresponding to the genes CYP1A-B, CYP2A-2M, CYP3A1, CYP4A-4S, CYP5, CYP6A-6W, CYP7A-B, CYP8, CYP8B, CYP9B, CYP9C, CYP9F, CYP9H, CYP10, CYP11, CYP11A-B, CYP12-12E, CYP13A, CYP17, CYP19, CYP19A, CYP18A, CYP21, CYP21A, CYP24, CYP26, CYP26A, CYP27A-27BB, CYP28A-28D, CYP36A, CYP39, CYP44, CYP46, CYP49A, CYP51, CYP52A, CYP53A, CYP55A, CYP56, CYP57A, CYP58, CYP59A, CYP60A-60B, CYP61, CYP62, CYP64, CYP65A, CYP67, CYP71A, CYP71B, CYP71C, CYP71D, CYP71E, CYP73A, CYP74A CYP75A, CYP76A-76C, CYP77A, CYP78A, CYP79A-79B, CYP80A-80E, CYP82A, CYP83A-83B, CYP84A, CYP85, CYP86A, CYP88A, CYP89A, CYP90A-90C, CYP91A, CYP93A-93B, CYP94A, CYP97B, CYP98A, CYP99A, CYP101, CYP102A, CYP103, CYP104, CYP105A-105E, CYP106, CYP106A, CYP107A-107J, CYP108, CYP109, CYP110, CYP112, CYP112A, CYP113A, CYP114, CYP116-117, CYP117A, CYP119-126, CYP127A, CYP128-132, CYP133B, CYP134, CYP135A, CYP135B, CYP136-144, CYP152A, CYP301A, CYP302A, CYP303A, CYP304A, CYP305A, CYP306A, CYP307A, CYP308A, CYP309A, CYP310A, CYP311A, CYP312A, CYP313A-313B, CYP314A, CYP315A, CYP316A, CYP317A, CYP318A and their subfamilies and subgroups; in particular, it is desirable to use one or a mixture of two or more enzyme proteins selected from the those corresponding to the genes CYP1A1, CYP1A2, CYP1B1, CYP2A6, CYP2B6, CYP2C8, CYP2C91, CYP2C92, CYP2C93, CYP2C18, CYP2C19, CYP2D61, CYP2D610, CYP2E1, CYP3A4, CYP3A5, CYP3A7, CYP4A11, CYP4F2, CYP4F3A and CYP19.

The drug-metabolizing enzyme covered by the present invention may be used in the form of a protein with purified activity, or in some cases it may be used in the form of a partially purified, roughly purified or unpurified extract of animal or plant tissue or microorganisms.

In the present invention, desirable combinations of drug-metabolizing enzyme with the substrate and specific antibody used in reacting it include for example human CYP19 with testosterone and anti-testosterone antibodies, human CYP19 with androstenedione and anti-estrone antibodies, human CYP3A with progesterone and anti-6β-hydroxyprogesterone, human CYP21A with progesterone and anti-11-deoxycorticosterone antibodies, human CYP1A2 with (R)-warfarin and anti-hydroxywarfarin antibodies, (human CYP2C8 or human CYP2C9 or human CYP2C19) with dibenzylfluorescein and anti-fluorescein antibodies, and human 17β-hydroxysteroid dehydrogenase with estrone and anti-estradiol antibodies. In some cases it will probably be necessary to consider differences in the substrate specificity of the drug-metabolizing enzyme depending on the species or on the type of single nucleotide polymorphism in humans.

Enzyme immunoassay, surface plasmon resonance, micro-differential thermal analysis or quartz resonance using specific antibodies to the converted product by the action of the drug-metabolizing enzyme may be selected as the immunochemical measurement method of the present invention. In the case of enzyme immunoassay, it is possible to use either a competitive method in which the product is bound to an enzyme as a hapten and the enzyme-labeled product combined with a solid-phase product-specific antibody, or else a competitive method in which the product is cross-linked with bovine serum albumin or the like and immobilized, and combined with enzyme-labeled product-specific antibody. These methods may also be modified as necessary for purposes of use. In such competitive enzyme immunoassay methods, the greater the amount of product resulting from conversion by the drug-metabolizing enzyme, the smaller the amount of enzyme-labeled product or antibody that is ultimately detected, and hence the smaller the resulting signal. When surface plasmon resonance is used, product-specific antibodies or product cross-linked with bovine serum albumin or the like may be immobilized on the surface of the gold thin-film to be used. In the case of micro-differential thermal measurement, the small amount of heat generated during the reaction of product and antibody can be measured directly. In the case of quartz resonance, product-specific antibodies or the product cross-linked with bovine serum albumin or the like may be immobilized on a quartz oscillator for purposes of use.

Antibodies used in the immunochemical measurements of the present invention are all classes of immunoglobulin, namely IgG, IgM, IgE, IgA and IgD, and fish, amphibian, reptile or mammalian antibodies may be used. They may be polyclonal antibodies obtained from the bodily fluids of such animals, monoclonal antibodies produced from hybridomas, or antibodies manufactured by genetic engineering. The antibodies may be whole molecules, or they may be any form of fragments such as Fab, (Fab)₂, Fab', Fv, scFv or minibodies as long as activity of the binding area is maintained. These antibodies need to have high specificity for the product of the drug-metabolizing enzyme during measurement. Use of an antibody which is cross-reactive with a number of chemical substances is within the scope of the present invention, but in that case there will probably be limits on the types of chemical substances to be evaluated

The unmodified endogenous substrate of the present invention is a steroid hormone or other chemical substance naturally present in the body, and it is a substrate which has not had any functional groups artificially introduced or removed. However, modified substances such as sulfuric acid conjugates and glucuronic acid conjugates which may be present in the body can be used.

In the present invention, products of conversion by the drug-metabolizing enzyme may be analyzed and quantified by gas chromatography (GC), mass spectrometry (MS), high-performance liquid chromatography (HPLC), NMR or immunochemical measurement methods. Before analysis and quantification, the reaction liquid containing the products may be treated with resins having various functional groups or subjected to organic solvent extraction, solid-phase extraction, silica gel chromatography or the like. Immunochemical measurement is desirable since it does not require protein removal or separation prior to analysis and allows a number of samples to be analyzed simultaneously.

Antibodies used in the immunochemical measurements of the present invention may be immobilized for use on an insoluble carrier in the case of enzyme immunoassay, surface plasmon resonance or quartz resonance, or they may be used in solution without fixing. Antibodies used in micro-differential thermal analysis do not need to be immobilized. When antibodies used in enzyme immunoassay, surface plasmon resonance and quartz resonance are used in solution without being immobilized on an insoluble carrier, the protein (product-BSA conjugate or the like) with the cross-linked product needs to be fixed on an insoluble carrier.

Antibodies used in the immunochemical measurements of the present invention are any class of immunoglobulin, namely IgG, IgM, IgE, IgA and IgD, and may be fish, amphibian, reptile or mammalian antibodies. The antibodies may be polyclonal antibodies derived from the body fluids of such animals, monoclonal antibodies produced by hybridomas or antibodies manufactured by genetic engineering. The antibodies may be whole molecules or they may be any form of fragments such as Fab, (Fab)₂, Fab', Fv, scFv or minibodies as long as activity of the binding area is maintained. These antibodies need to have high specificity for the product of the drug-metabolizing enzyme during measurement.

"High specificity" here signifies that reactivity with groups of chemical substances having structural similarity to the substrate compound is less than one in a thousand or preferably less than one in ten thousand. The lower the reactivity the better. It is preferably that there be multiple chemical groups and multiple types of groups with structural similarity. Reactivity may be evaluated for example by ordinary methods of competitive enzyme immunoassay.

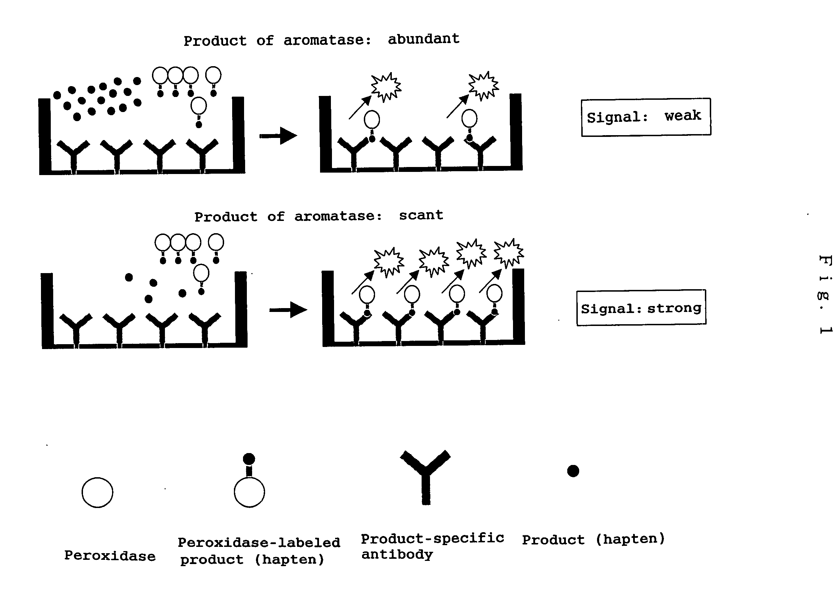

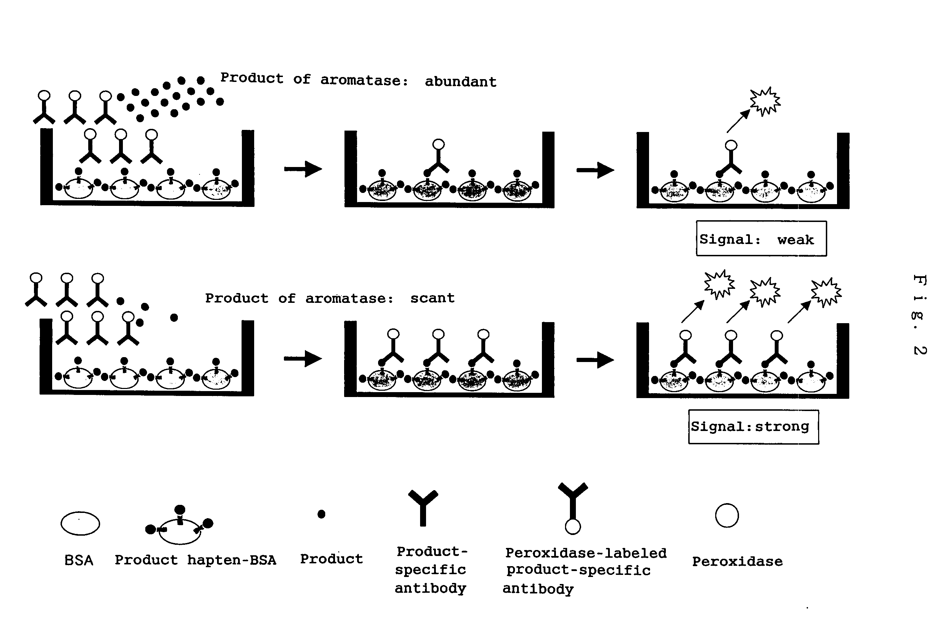

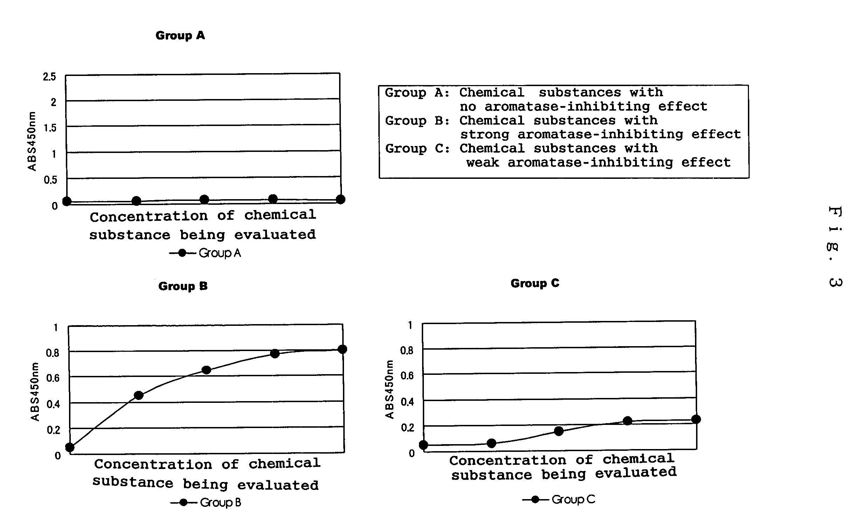

As used here "competitive method" signifies a measurement system in which a labeled product A (A-L) which is the target of specific antibody binding is reacted competitively with (A) produced by drug-metabolizing enzyme on a carrier on which A-specific antibody (Ig) has been immobilized, and the amount of the label (L) of free (A-L) or (A-L) bound to the solid phase is measured. An example of a competitive measurement system using an enzyme (peroxidase) as the label is shown in Figure 1. Alternatively, as shown in Figure 2, a complex (A-B) of A cross-linked with a protein (B) which does not produce a signal may be immobilized on a carrier, and labeled antibody (Ig-L) reacted competitively with (A) produced by drug-metabolizing enzyme. In this case the amount of the label (L) of free (Ig-L) or (Ig-L) bound to the carrier is measured. A model of a dose-response curve for inhibition of drug-metabolizing enzyme obtained from a measurement system using an enzyme labeled product and solid-phase specific antibodies is shown in Figure 3.

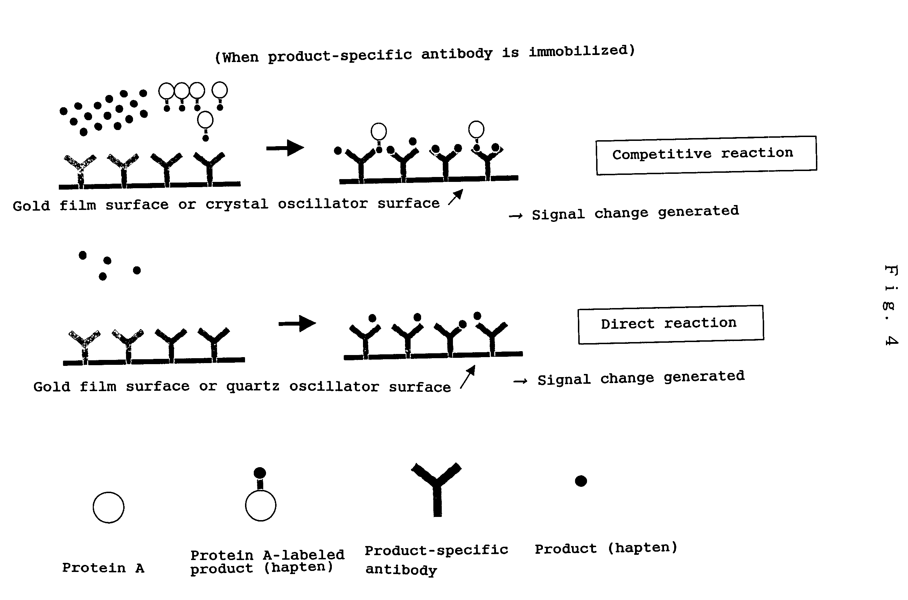

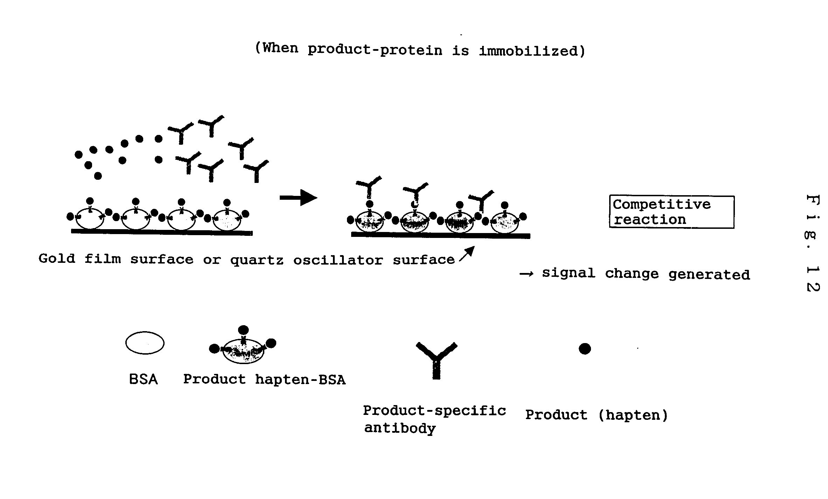

The measurement principles when using surface plasmon resonance or quartz resonance in the present invention are shown in Figures 4 and 12. When using these methods, the specific antibodies are either immobilized on a gold thin-film surface or a crystal resonator, or else a conjugate (A-B) of protein (A) with a protein (B) which does not produce a signal is immobilized on a gold thin-film surface or crystal oscillator.

Examples of the insoluble carrier used in fixing the product-specific antibody or cross-linked product-protein conjugate of the present invention include polystyrene, nylon, polycarbonate and other plastics, agarose, cellulose, polyacrylamide, dextran, sepharose, nitrocellulose, glass, filter paper and the like. Various functional groups (hydrazide, alkylamino, amino, hydroxyl and carboxyl groups and the like) may be introduced into the carriers. The carrier may be used in a convenient form such as a microtiter plate, film, sheet or beads. If is preferable that the adsorbancy and binding power of the insoluble carrier be confirmed in advance, but this information is not always necessary. Coupling by covalent binding is also possible when preparing a solid-phase carrier in the present invention. Glutaraldehyde, carbodiimide or the like may be used as the coupling reagent.

The base buffer of the adsorption liquid used in immobilizing the antibody on solid phase or cross-linked product-protein conjugate of the present invention may be an ordinarily used buffer such as 10 mM phosphoric acid buffer (containing 150 mM NaCl, pH 7.2), 50 mM carbonic acid buffer (pH 9.6), 10 mM tris-hydrochloric acid buffer (containing 150 mM NaCl, pH 8.5) or the like, within the pH range (pH 3.0-10.0 or preferably pH 5.0-9.0) at which the stability of the antibody or cross-linked product-protein conjugate is maintained. The type of salt and buffer concentration are not limited by the examples given, but can be varied as necessary, and depending on the type of antibody solid phase or cross-linked product-protein conjugate it is possible to add KCl, MgCl₂, MnCl₂ or trace amounts of heavy metal salts within the range of 0.01-300 mM. Following the adsorption and binding reactions, serum albumin, casein, gelatin or the like can be used in the range of 0.01%-10% (W/V) as a blocking agent to prevent non-specific reactions. Base buffers that can be used for this blocking agent including the phosphoric acid and tris-hydrochloric acid buffers used in the adsorption liquid as well as maleic acid, lactic acid and other organic acid buffers in the range of 10-300 mM. The pH can be selected from the optimal range for stability of the receptors (including antibodies) involved in the measurement system, and should generally be between 5.0 and 8.5. Saccharose, glucose, dextran and other sugars may also be added as stabilizers in the range of 0.01-20% (W/V) for purposes of stabilization after fixing.

A concentration in the range of 0.1 pg (picograms)/ml to 1 mg/ml can be used for immobilizing the antibody or cross-linked product-protein conjugate involved in the measurement system. A range of 10 pg/ml to 50 µg/ml or more preferably 0.1 µg/ml to 10 µg/ml is preferred.

The time required for adsorption and binding may be selected within the range of 5 minutes to 72 hours, and preferably in the range of 1 to 36 hours. The incubation temperature may be selected in the range of 1°C to 45°C or more preferably 2°C to 37°C.

After adsorption by the insoluble carrier of the antibody or cross-linked product-protein conjugate in the present invention, they can be washed as necessary in a 10 mM phosphoric acid buffer (pH 7.2, containing 150 mM NaCl) or other wash liquid. A mild nonionic surfactant such as Tween 20 or Triton X-100 may also be added as necessary as a cleaning agent.

Substrates that can be used for measuring drug-metabolizing enzyme activity include for example testosterone, dihydrotestosterone, androstendione, 7-ethoxy-3-cyanocoumarin, 7-methoxy-4-trifluoromethylcoumarin, 7-benzyloxy-4-trifluoromethylcoumarin, dibenzylfluorescein or 7-ethoxy-4-trifluoromethylcoumarin in the case of CYP19. For CYP1A2, aminophylline, amitriptyline, betaxolol, caffeine, clomipramine, clozapine, chlorpromazine, fluvoxamine, haloperidol, imipramine, metoclopramide, olanzapine, ondansetron, propranolol, tacrine, theophylline, thioridazine, trifluoperazine, verapamil, (R)-warfarine and the like can be used. For CYP2C9, amitriptyline, cerivastatin, dicofenac, fluoxetine, flavastatin, ibuprofen, losartan, neproxen, tolbutamide, torsemide, (S)-warfarin and the like can be used. For CYP2C19, amitriptyline, citalopram, clomipramine, diazepam, flunitrazepam, imipramine, lansoprazole, omeprazole and the like can be used. For CYP2D6, aminoptirin, betaxolol, clomipramine, clozapine, codeine, desipramine, dextromethorphan, donepazil, flecainide, fluoxetine, haloperidol, imipramine, methadone, metoclopramide, metoprolol, mexiletine, nortriptyline, olanzapine, ondansetron, orphenadrine, paraxetin, pindolol, propafenone, propranol, risperdone, sertraline, thioridazine, timolol, trazodone, venlafaxine and the like are used. For CYP2E1, acetaminophen, caffeine, chlorzoxazone, dextromethorphan, ethanol, theophylline, venlafaxine and the like can be used. for CYP3A, alaprazolam, amiodarone, amitriptyline, astemizole, budesonide, bupropin, buspirone, caffein, carbamazepine, cerivastatin, cisapride, clarithomycin, clomipramine, clonazepam, codeine, cyclosporin, dexamethasone, dextromethophan, dihydroepiandrosterone (DHEA), diazepam, diltiazem, disopyramide, donepazil, doxycyline, erythromycin, estradiol, ethynylestradiol, felodipine, fluoxetine, imipramine, lansoprazole, lidocaine, loratadine, lovastatin, midazolam, nefazodone, nicardipine, nifedipine, nisoldipine, norethindrone, omeprazole, ondansetron, orphenadrine, paroxetine, progesterone, propafenone, quetiapine, quinidine, rifampin, sertraline, sibutramine, sildenafil, simvastatin, tacrolimus, tamoxifen, terfenamide, testosterone, theophylline, trazodone, triazolam, venlafaxine, verapamil, vinblastine, (R)-warfarin and zolpidem and the like can be used. For 17β-hydroxysteroid dehydrogenase, estrone and the like can be used. Many of these substrates are endogenous hormones or are used as drugs, and when they are used for evaluating drug-metabolizing enzyme inhibitors in the present invention, the evaluation will include the effects of drug-drug interaction as well as inhibitory effects on drug-metabolizing enzymes.

There are no limits on the antibodies used in measuring drug-metabolizing enzyme activity as long as they can specifically recognize the product of metabolization of the aforementioned substrate, and for example anti-testosterone antibodies, anti-estrone antibodies, anti-6β-hydroxyprogesterone antibodies, anti-11-deoxycorticosterone, anti-hydroxywarfarin antibodies, anti-fluorescein antibodies and the like can be used. It is better to use practical monoclonal antibodies which are highly sensitive to the metabolic product and cross-react little with the substrate.

When evaluating inhibition of drug-metabolizing enzyme activity, the drug-metabolizing enzyme may be trapped after the reaction at the time of the drug-metabolizing enzyme reaction. It may also be captured and fixed after the drug-metabolizing enzyme reaction by drug-metabolizing enzyme antibodies immobilized on an insoluble carrier, and removed from the reaction system. The drug-metabolizing enzyme can also be biotinized in advance, and then removed from the reaction system after the drug-metabolizing enzyme reaction using streptoavidin immobilized on an insoluble carrier. Alternatively, when evaluating inhibitory effects on drug-metabolizing enzyme activity, heat treatment can be applied to suppress activity of the drug-metabolizing enzyme following the reaction, or else a known drug-metabolizing enzyme inhibitor can be added after the reaction.

There are no limitations on the regenerating system for supplying the nicotinamide-adenine dinucleotide phosphate (reduced form, sometimes referred to below as "NADPH") required by drug-metabolizing enzyme reactions and cytochrome P450 reactions in particular, as long as it is an enzyme system capable of producing NADPH, but preferably the system should not affect the drug-metabolizing enzyme reaction or the subsequent immune reaction. Such enzyme systems include combinations of glucose-6-phosphate, nicotinamide-adenine dinucleotide phosphate (oxidized form, sometimes referred to below as "NADP⁺") and glucose-6-phosphate dehydrogenase, or isocitric acid, NADP⁺ and isocitric acid dehydrogenase.

When the substrate of the drug-metabolizing enzyme reaction is present in extremely large quantities, the immunochemical reaction following the drug-metabolizing enzyme reaction may be affected. Consequently, the amount of substrate should be adjusted to a level at which there is practically no effect. "Practically no effect" signifies an effect of 10% or of the signal of the reference value (0 concentration of the chemical substance to be evaluated) of the standard curve for the immunoassay reaction. More specifically, using cytochrome P450 CYP19 for example and testosterone as the substrate, anti-17β-estradiol antibodies as the specific antibodies and 17β-estradiol-labeled peroxidase as the standard substance, even if the anti-17β-estradiol antibodies are extremely specific and have very little cross-reactivity with testosterone, at the substrate concentration (1-100 mM) normally used for drug-metabolizing enzyme reactions, the amount is far to great for the specific antibodies, and the immune reaction of the anti-17β-estradiol antibodies with the 17β-estradiol may be affected. It is possible to effectively eliminate the effect on the immune reaction by reducing the concentration of the testosterone which is the substrate. Substances of other P450 enzymes are similar. In some cases concentrations of other androgen substrates may be preferably in the range of 0.05 nM to 500 µM, depending on the nature of the product-specific antibodies. In the case of cytochrome P450 CYP2C19, if dibenzylfluorescein is selected as the substrate, anti-fluorescein antibodies as the specific antibodies and fluorescein isocyanate-labeled peroxidase as the labeled product, even if the anti-fluorescein antibodies are extremely specific and there is very little cross-reactivity with the dibenzylfluorescein, at the substrate concentration (1-100 mM) normally used for drug-metabolizing enzyme reactions, the amount is far to great for the specific antibodies, and the immune reaction of the anti-fluorescein antibodies with the fluorescein may be affected. It is possible to effectively eliminate the effect on the immune reaction by reducing the concentration of the dibenzylfluorescein which is the substrate. Depending on the nature of the antibodies, the substrate concentration for the cytochrome P450 reaction in some cases needs to be 0.05 nM-500 µM.

When using NADPH as another electron transporter in a drug-metabolizing enzyme reaction and particularly cytochrome P450 reaction, it can be added in NADPH form directly to the reaction system in the normal range of 0.1-10.0 mM. It can also be added as NADP⁺, and the recovery system combinations described above can also be added. A combination of glucose-6-phosphate, NADP⁺ and glucose-6-phosphate dehydrogenase can be used in the range of 0.2-20 mM, 0.1-5.0 mM and 0.1-20 units/ml. A range of 0.5-10 mM, 0.5-3.0 mM and 0.5-10 units/ml can be used by preference. Glucose-6-phosphate, NADP⁺ and NADPH can be used in the form of sodium or potassium salts or the like, while the enzymes used may be highly purified products, partially purified products, ammonium sulfate suspensions or the like.

When enzyme immunoassay is used for immunochemical measurement, the labeling enzyme may be any enzyme normally used in enzyme immunoassay, such as peroxidase, alkaliphosphatase, glucose oxidase, β-galactosidase, urease, lysozyme or the like without limitation. According to the enzyme selected, substrates such as tetramethylbenzidine (TMB), o-phenylenediamine, 2,2-azinodi-(3-ethylbenzothiazoline-6-sulfonic acid)diammonium (ABTS), p-nitrophenylphosphoric acid, o-nitrophenyl-β-D-galactoside and the like which are matched to the various enzymes can be used.

In the drug-metabolizing enzyme and immunochemical reactions, organic solvents such as dimethylsulfoxide and dimethylformamide may be included to enhance the solubility of the chemical substance to be evaluated. Such organic solvents may be used within the range at which their effect on the cytochrome P450 and immunochemical reactions is none or negligible. Depending on the type of cytochrome P450, dimethylsulfoxide and dimethylformamide can be used at levels of 0.1-20% or preferably 0.2-10% or more preferably 0.2-5%.

The measurement kit described in the present invention is a group of various reagents (including solid-phase carriers) which allow measurement by enzyme immunoassay, surface plasmon resonance, micro-differential thermal measurement or quartz resonance using antibodies which specifically recognize the aforementioned product. More specifically, it is a set combining ingredients selected as needed from the following: plates immobilizing specific antibodies (or products cross-linked with protein), gold thin-film chips immobilizing specific antibodies (or products cross-linked with proteins), quartz resonators fixed with specific antibodies (or product cross-linked proteins), reaction buffers, specific antibody liquids, antibody sample diluents, standard substances, enzyme substrate liquids for measuring the amount of label and the like.

In the present invention, "non-steroid chemical substances" refers to chemical substances such as aminoglutethimides and naphthoflavones that lack a steroid structure. Because some chemical substances with steroid structures are structurally similar to the products of drug-metabolizing enzymes and cytochrome P450 in particular, they may be somewhat cross-reactive with product-specific antibodies in some cases. The resulting inhibitory effect will then be underestimated, and there is a possibility of false negatives. When evaluating such chemical substances, it is necessary to compare the results with those from a measurement system from which only the drug-metabolizing enzyme has been removed, and consider the inhibitory effect of the chemical substance. For various reasons, however, some of the cytochrome P450 inhibitors which have been developed as drugs lack a steroid structure, and the present invention is particularly useful for screening non-steroid cytochrome P450 inhibitors. Moreover, most chemical substances which cause problems as endocrine disruptors have aromatic rings but many are non-steroidal, so the present invention is especially useful for screening endocrine disrupting chemical substances.

Further, the measuring method of the invention can be carried out manually or automatically such as robotics. Furthermore, the amount of reagents used can be suitably selected according to a operation method. The method of the invention can be carried out not only in a usual scale from µl to mL, but also in a downsized scale.

### BEST MODE FOR CARRYING OUT THE INVENTION

Preferred embodiments for carrying out the present invention are described below as examples.

However, the present invention is of course not limited by these embodiments.

### [Example 1A]

### (Preparation of a plate immobilizing anti-estradiol antibodies)

Purified anti-estradiol antibodies obtained using as the antigen a protein formed by cross-linking using the third position of estradiol were diluted in 50 mM carbonic acid buffer (pH 9.6) to a concentration of 1 µg/ml (antibody adsorption liquid), and 100 µL per well was poured into Corning Costar microtiter plates (polystyrene 96-well type). The surfaces were then sealed, and the plates left overnight at 4°C. The following day the antibody adsorption liquid was removed, and each well washed 3 times with 200 µL of 10 mM phosphoric acid buffer (pH 7.2, containing 150 mM NaCl). After washing, using 10 mM phosphoric acid buffer (pH 7.2, containing 150 mM NaCl: phosphoric acid buffer 1) as the base, each well was filled with 200 µL of a blocking solution containing 0.2% casein and 10% sucrose. After being filled with the blocking liquid the plates were left overnight at 4°C, and the following day the blocking liquid was removed and vacuum drying applied for 16 hours. The vacuum-dried plates were sealed in storage bags under reduced pressure, and stored at 4°C prior to the following measurements.

### (Measurement of cytochrome P450)

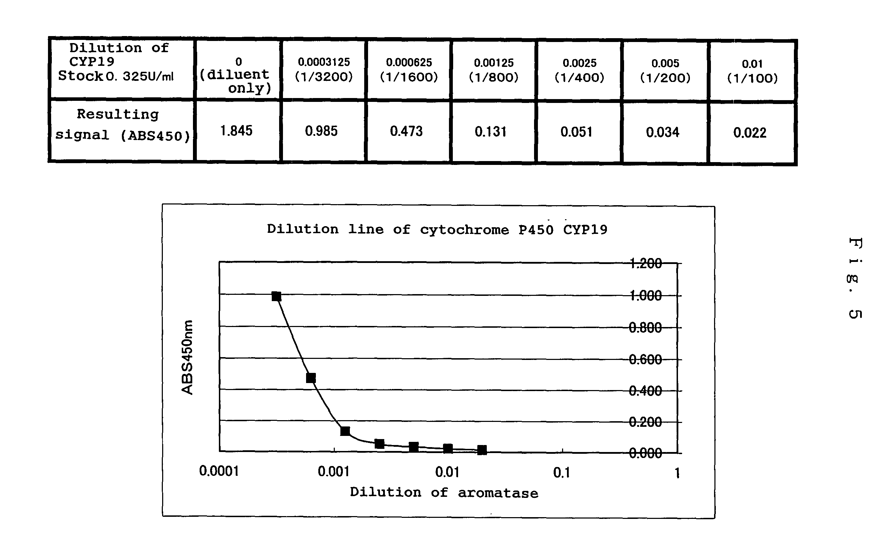

Recombinant human cytochrome P450 CYP19 from Gentest Co. was diluted with 100 mM phosphoric acid buffer (pH 7.2, containing 0.1% BSA: phosphoric acid buffer 2), and a concentration sequence prepared. 25 µL of each concentration and 100 µL of reaction mixtures with phosphoric acid buffer 2 as the base containing 0.5 units/ml of glycerol-6-phosphoric acid dehydrogenase (sometimes described below as "G6PDH"), 4.13 mM MgCl₂, 1.63 mM NADP 2Na, 4.13 mM G6P Na and 62.5 µM testosterone was mixed in polypropylene assay plates (96-well) together with 25 µL of phosphoric acid buffer 2, and reacted for 20 minutes at 37°C. cytochrome P450 CYP19 converts testosterone to 17β-estradiol. 50 µL of this reaction liquid was taken, mixed together with 50 µL of peroxidase-labeled estradiol (E2-HRP) having 10 mM phosphoric acid buffer as the base (pH 7.2, containing 0.1% BSA: phosphoric acid buffer 3) on plates immobilizing anti-estradiol antibodies, and reacted for 1 hour at 4°C. Following the reaction the plates were washed three times with 200 µL of phosphoric acid buffer 1 containing 0.1% Tween 20, and 100 µL of an enzyme reaction solution containing tetramethylbenzidine was added and incubated for 20 minutes at 37°C. Next the enzyme reaction was halted with 100 µL of 1N sulfuric acid, and absorbance was measured at 450 nm using a microplate reader, and the data processed. The relationship between the resulting absorbance and the concentration sequence of human aromatase is shown in Figure 5. There is an extremely high correlation between absorbance and aromatase concentration, indicating that using the method of this example it is possible to measure cytochrome P450 CYP19 activity in a sample from absorbance data.

### [Example 2A]

### (Evaluation (1) of the inhibitory effect of a chemical substance on cytochrome P450 CYP19)

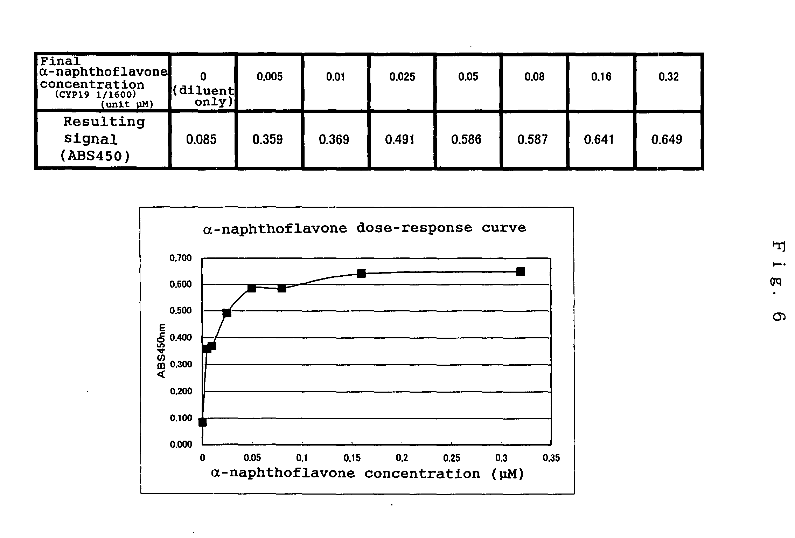

Human cytochrome P450 CYP19 from BD Science was diluted about 1600 times with phosphoric acid buffer 2, and 25 µL of this human cytochrome P450 CYP19 solution and 100 µL of a reaction mixture with phosphoric acid buffer 2 as the base containing 0.5 units/ml G6PDH, 4.13 mM MgCl₂, 1.63 mM NADP 2Na, 4.13 mM G6P Na and 62.5 µM testosterone, together with 25 µL of an α-naphthoflavone 0 µM-3.2 µM (final concentration, n=6, 8 levels, total 48 samples) solution with phosphoric acid buffer 2 as the base, was mixed in polypropylene assay plates (96-well) and reacted for 20 minutes at 37°C. 50 µL of this reaction liquid was taken and treated as in Example 1A above, and the relationship between α-naphthoflavone solution concentration and absorbance at 450 nm examined. The correspondences are shown in Figure 6. The higher the concentration of α-naphthoflavone, the greater the inhibition of cytochrome P450 CYP19, with less estradiol produced and the signal strength increasing in the enzyme immunoassay method.

### [Example 3A]

### (Evaluation (2) of the inhibitory effect of a chemical substance on cytochrome P450 CYP19)

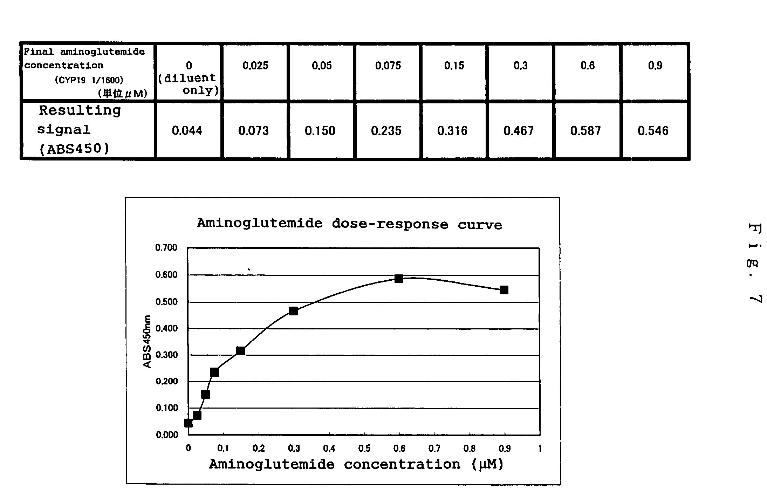

The same operations were performed as in Example 2A using 0-9 µM (final concentration, n=8, 8 levels, total 64 samples) of aminoglutethimide solution in place of α-naphthoflavone, and the relationship between aminoglutethimide solution concentration and absorbance at 450 nm was examined. The correspondences are shown in Figure 7. As in Example 2A, he higher the amount of aminoglutethimide the stronger the inhibition of cytochrome P450 CYP19, with less estradiol produced and the signal strength increasing in the enzyme immunoassay method.

### [Example 4A]

### (Evaluation of the inhibitory effect of a chemical substance on 17β-hydroxysteroid dehydrogenase)

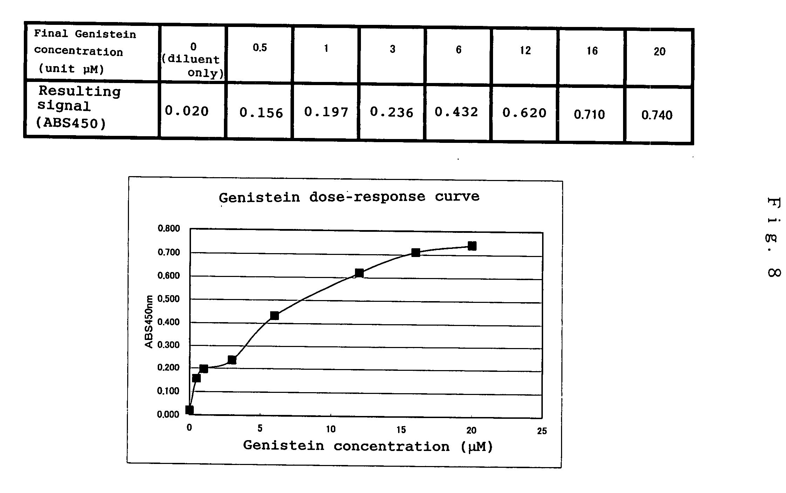

The same operations were performed as in Example 2A using Toyobo Co. *Pseudomonas*-derived 17β-hydroxysteroid dehydrogenase in place of cytochrome CYP19, estrone in place of testosterone as the substrate, and a 0-20 µM (final concentration) genistein solution in place of α-naphthoflavone, and the relationship between solution concentration and absorbance at 450 nm was examined. 17β-hydroxysteroid dehydrogenase converts estrone to 17β-estradiol. The correspondences are shown in Figure 8. As in Example 2A, the higher the amount of aminoglutethimide the stronger the inhibition of 17β-hydroxysteroid dehydrogenase, with less estradiol produced and the signal strength increasing in the enzyme immunoassay method.

### [Example 5A]

### (Preparation of plates immobilizing anti-fluorescein antibodies)

Purified anti-fluorescein antibodies obtained using as the antigen a protein cross-linked using the X position of fluorescein were diluted with 50 mM carbonic acid buffer (pH 9.6) to a concentration of 1 µg/ml (antibody adsorption liquid), and 100 µL per well was poured into Corning Costar microtiter plates (polystyrene 96-well type). The surfaces were then sealed, and the plates left overnight at 4° C. The following day the antibody adsorption liquid was removed, and each well washed three times with 200 µL of a 10 mM phosphoric acid buffer (pH 7.2, containing 150 mM NaCl). After washing, using 10 mM phosphoric acid buffer (pH 7.2, containing 150 mM NaCl) as the base, each well was filled with 200 µL of a blocking solution containing 0.2% casein and 10% sucrose. After being filled with the blocking liquid the plates were left overnight at 4°C, and the following day the blocking liquid was removed and vacuum drying applied for 16 hours. The vacuum-dried plates were sealed in storage bags under reduced pressure, and stored at 4°C prior to the following measurements.

### (Measurement of human cytochrome P450 CYP2C19)

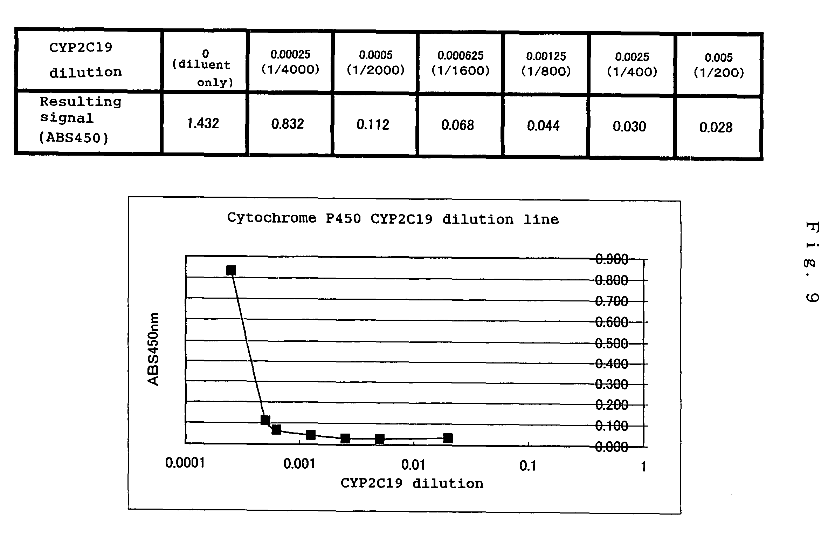

Recombinant human cytochrome P450 CYP19 from BD Science was diluted with 100 mM phosphoric acid buffer (pH 7.2, containing 0.1% BSA: phosphoric acid buffer 2), and a concentration sequence prepared. 25 µL of each concentration together with 100 µL of a reaction mixture with phosphoric acid buffer 2 as the base containing G6PDH (0.5 units/ml), 4.13 mM MgCl₂, 1.63 mM NADP 2Na, 4.13 mM G6P Na and 62.5 µM of dibenzylfluorescein (from BD Scinece) was mixed in polypropylene assay plates (96-well) together with 25 µL of phosphoric acid buffer 2, and reacted for 20 minutes at 37° C. 50 µL of this reaction liquid was taken, mixed together with 50 µL of peroxidase-labeled fluorescein (E2-HRP) from Sigma Co. having 10 mM phosphoric acid buffer as the base (pH 7.2, containing 0.1% BSA: phosphoric acid buffer 3) on anti-fluorescein antibody-coated plates from Cosmo Bio Co., and reacted for 1 hour at 4° C. Following the reaction the plates were washed three times with 200 µL of phosphoric acid buffer 1 containing 0.1% Tween 20, and 100 µL of an enzyme reaction solution containing tetramethylbenzidine was added and incubated for 20 minutes at 37° C. Next the enzyme reaction was halted with 100 µL of 1N sulfuric acid, and absorbance was measured at 450 nm using a microplate reader, and the data processed. The relationship between the resulting absorbance and the concentration sequence of human cytochrome P450 CYP2C19 is shown in Figure 9. There is an extremely high correlation between absorbance and human cytochrome P450 CYP2C19 concentration, indicating that using the method of this example it is possible to measure cytochrome P450 CYP19 activity in a sample from absorbance data.

### [Example 6A]

### (Evaluation (1) of the inhibitory effect of a chemical substance on human cytochrome P450 CYP2C19)

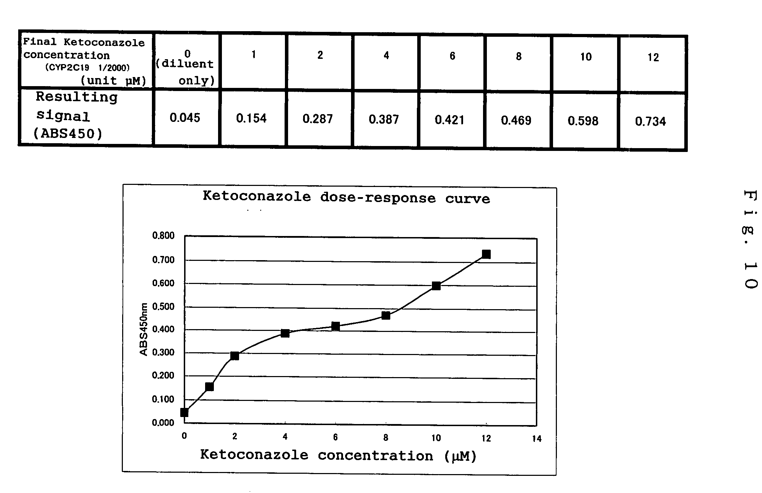

Recombinant human cytochrome P450 CYP2C19 from BD Science was diluted about 2000 times with phosphoric acid buffer 2, and 25 µL of this human cytochrome P450 CYP2C19 solution and 100 µL of a reaction mixture with phosphoric acid buffer 2 as the base and containing G6PDH (0.5 units/ml), 4.13 mM MgCl₂, 1.63 mM NADP 2Na, 4.13 mM G6P Na and 30 µM dibenzylflorescein, together with 25 µL a 0-12 µM (final concentration) solution of Sigma Co. ketoconazole with phosphoric acid buffer 2 as the base, was mixed in polypropylene assay plates (96-well) and reacted for 20 minutes at 37°C. 50 µL of this reaction liquid was taken and treated as in Example 1A above, and the relationship between ketoconazole solution concentration and absorbance at 450 nm examined. The correspondences are shown in Figure 10. The greater the amount of ketoconazole, the greater the inhibition of cytochrome P450 CYP2C19, with less fluorescein produced and the signal strength increasing in the enzyme immunoassay method.

### [Example 7A]

### (Evaluation (2) of the inhibitory effect of a chemical substance on human cytochrome P450 CYP2C19)

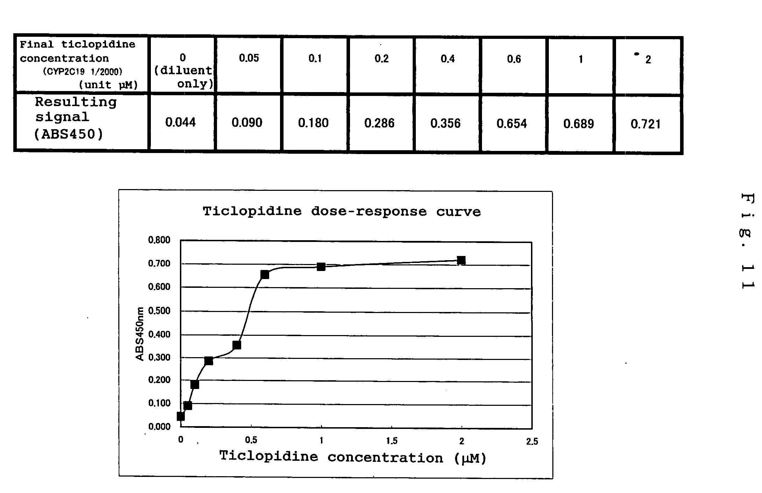

The same operations as in Example 2A were performed with 0-2 µM (final concentration) solutions of ticlopidine in place of ketoconazole, and the relationship between ticlopidine concentration and absorbance at 450 nm examined. The correspondences are shown in Figure 11. As in Example 2A, the greater the amount of ticlopidine, the greater the inhibition of human cytochrome P450 CYP2C19, with less fluorescein produced and the signal strength increasing in the enzyme immunoassay method.

### [Example 8A]

### (Specimen processing capability)

10 levels, 48 levels and 96 levels of known concentrations of α-naphthoflavone were prepared in place of the α-naphthoflavone dilution sequence of Example 2A, and aromatase reactions were performed in 96-well polypropylene microplates with n=2 for each, and immune reactions and coloring reactions performed by hand in antibody-coated 96-well polystyrene microplates. Total required times were about 2 hours, 2 hours and 2 hours 15 minutes, respectively. The time difference was due mainly to a difference in the number of plates to be washed and to the timing of the immune and enzyme reactions. It appears that different levels of samples can be measured efficiently in about 2 hours.

### [Example 1B]

### (Preparation of plates immobilizing anti-estradiol antibodies)

Purified anti-estradiol antibodies obtained using as the antigen a protein cross-linked using the 3^{rd} position of estradiol were diluted in 50 mM carbonic acid buffer (pH 9.6) to a concentration of 1 µg/ml (antibody adsorption liquid), and 100 µL per well was poured into Corning Costar microtiter plates (polystyrene 96-well type). The surfaces were then sealed, and the plates left overnight at 4° C. The following day the antibody adsorption liquid was removed, and each well washed 3 times with 200 µL of 10 mM phosphoric acid buffer (pH 7.2, containing 150 mM NaCl). After washing, using 10 mM phosphoric acid buffer (pH 7.2, containing 150 mM NaCl: phosphoric acid buffer 1) as the base, each well was filled with 200 µL of a blocking solution containing 0.2% casein and 10% saccharose. After being filled with the blocking liquid the plates were left overnight at 4°C, and the following day the blocking liquid was removed and vacuum drying applied for 16 hours. The vacuum-dried plates were sealed in storage bags under reduced pressure, and stored at 4°C prior to the following measurements.

### (Preparation of human cytochrome P450)

A placental system expressing pig cytochrome P450 CYP19 was prepared according to the methods described in *Mol. Endocrinol. Vol. 16*, pp 1456-1468 (2002), and a mutation introduced replacing isoleucine 133 with methionine or alanine according to ordinary methods. Two enzyme preparations, pig cytochrome P450 CYP19 (133Ile) and (133Met), were prepared as described in the literature and used in the following tests.

### (Measurement of cytochrome P450)

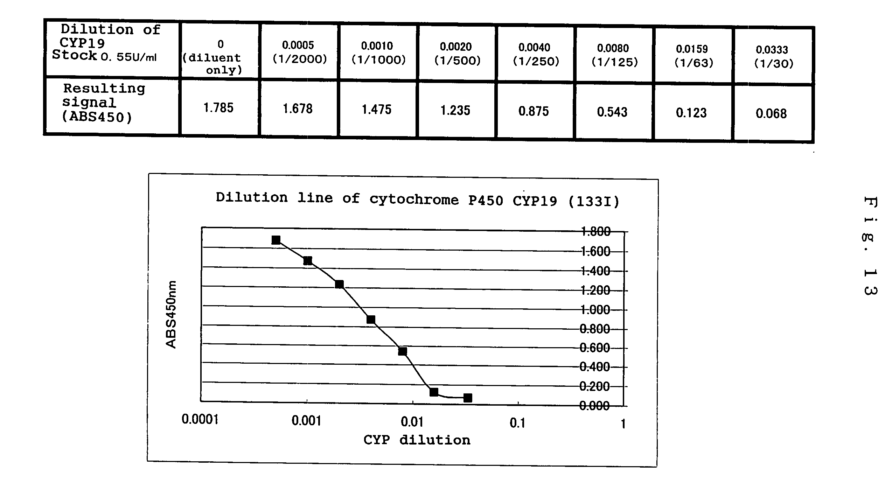

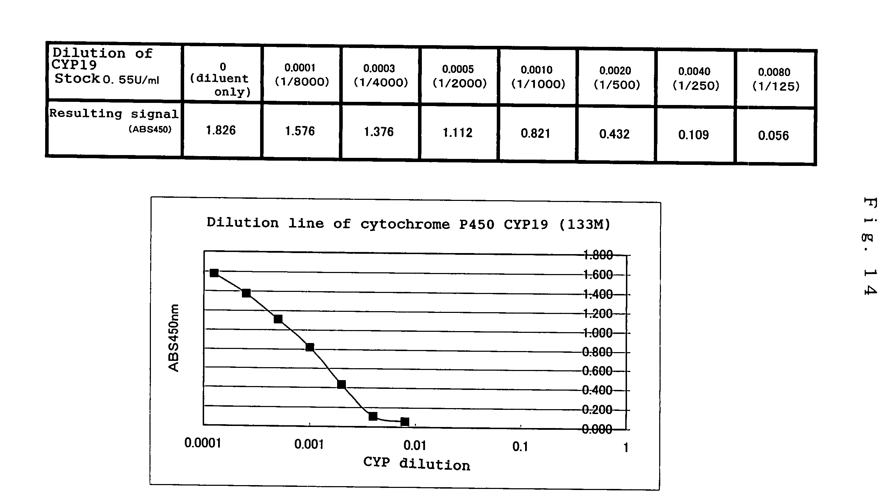

The two enzyme preparations, pig cytochrome P450 CYP19 (133Ile) and (133Met), were diluted with 100 mM phosphoric acid buffer (pH 7.2, containing 0.1% BSA: phosphoric acid buffer 2), and a concentration sequence prepared. 25 µL of each concentration and 100 µL of a reaction mixture with phosphoric acid buffer 2 as the base containing glycerol-6-phosphoric acid dehydrogenase (referred to below as "G6PDH") (0.5 units/ml), 4.13 mM MgCl₂, 1.63 mM NADP 2Na, 4.13 mM G6P Na and 62.5 µM testosterone was mixed in 96-well polpropylene assay plates together with 25 µL of phosphoric acid buffer 2, and reacted for 20 minutes at 37°C. Pig cytochrome P450 CYP19 can convert testosterone to 17β-estradiol. 50 µL of this reaction liquid was taken, mixed together with 50 µL of peroxidase-labeled estradiol (E2-HRP) having 10 mM phosphoric acid buffer as the base (pH 7.2, containing 0.1% BSA: phosphoric acid buffer 3) on plates immobilizing anti-estradiol antibodies, and reacted for 1 hour at 4°C. Following the reaction the plates were washed three times with 200 µL of phosphoric acid buffer 1 containing 0.1% Tween 20, and 100 µL of an enzyme reaction solution containing tetramethylbenzidine was added and incubated for 20 minutes at 37°C. Next the enzyme reaction was halted with 100 µL of 1N sulfuric acid, and absorbance measured at 450 nm using a microplate reader and the data processed. The relationship between the resulting absorbance and the concentration sequences of the three kinds of pig cytochrome P450 CYP19 is shown in Figures 13 and 14. There is an extremely high correlation between absorbance and enzyme concentration, indicating that using the method of this example it is possible to use absorbance data to measure the activity of three kinds of pig cytochrome P450 CYP19 in a sample.

### [Example 2B]

### (Evaluation of the inhibitory effect of a chemical substance on three kinds of pig cytochrome P450 CYP19)

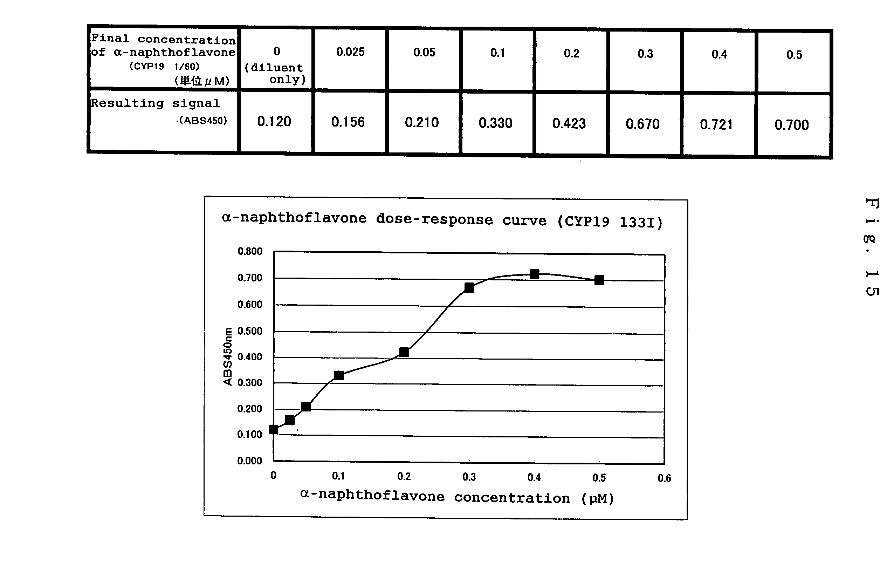

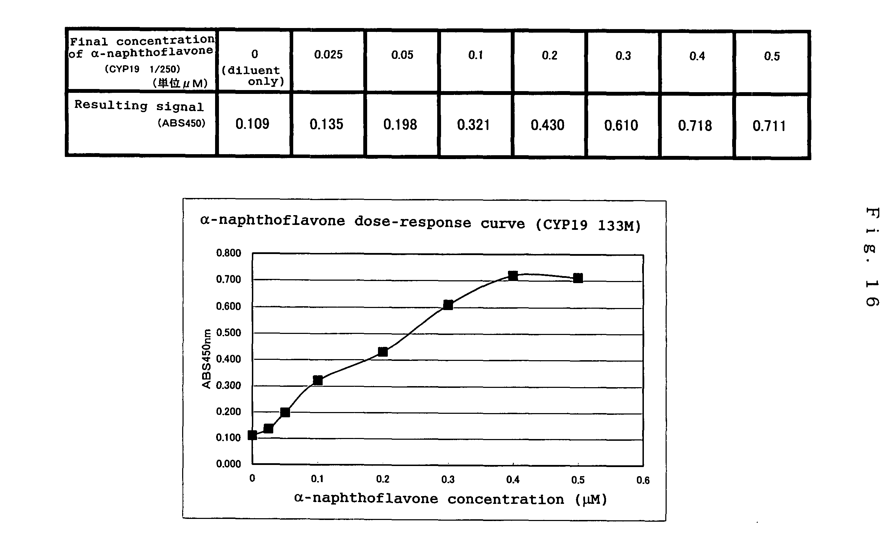

Pig cytochrome P450 CYP19 (133Ile) and (133Met) was diluted about 60 times and 250 times, respectively with phosphoric acid buffer 2, and 25 µL of each pig cytochrome P450 CYP19 solution and 100 µL of a reaction mixture with phosphoric acid buffer 2 as the base containing 0.5 units/ml G6PDH, 4.13 mM MgCl₂, 1.63 mM NADP 2Na, 4.13 mM G6P Na and 62.5 µM testosterone together with 25 µL of a solution of 0-3.2 µM (final concentration) Sigma Co. α-naphthoflavone having phosphoric buffer 2 as the base was mixed in 96-well polpropylene assay plates and reacted for 20 minutes at 37°C. 50 µL of this reaction liquid was taken and subjected to the same operations as in Example 1B, and the relationship between absorbance at 450 nm and etomidate solution concentration was examined. The correspondences are shown in Figures 15 and 16. The greater the amount of α-naphthoflavone, the greater the inhibition of pig cytochrome P450 CYP19, and the degree of inhibition differed greatly depending on whether preparation (133Ile) or preparation (133Met) was used.

### [Example 3B]

### (Preparation of plates immobilizing anti-fluorescein antibody)

Purified anti-fluorescein antibodies obtained using as the antigen a protein cross-linked using the X position of fluorescein were diluted with 50 mM carbonic acid buffer (pH 9.6) to a concentration of 1 µg/ml (antibody adsorption liquid), and 100 µL per well was poured into Corning Costar microtiter plates (polystyrene 96-well type). The surfaces were then sealed, and the plates left overnight at 4°C. The following day the antibody adsorption liquid was removed, and each well washed 3 times with 200 µL of 10 mM phosphoric acid buffer (pH 7.2, containing 150 mM NaCl). After washing, using 10 mM phosphoric acid buffer (pH 7.2, containing 150 mM NaCl: phosphoric acid buffer 1) as the base, each well was filled with 200 µL of a blocking solution containing 0.2% casein and 10% sucrose. After being filled with the blocking liquid the plates were left overnight at 4°C, and the following day the blocking liquid was removed and vacuum drying applied for 16 hours. The vacuum-dried plates were sealed in storage bags under reduced pressure, and stored at 4°C prior to the following measurements.

### (Preparation of human cytochrome P450)

A system expressing human cytochrome P450 CYP2C9 was constructed according to the methods of Goldstein et al (Goldstein J.A. et al, *Biochemistry Vol. 33* pp 1743-1752 (1994)). A single nucleotide mutation (A→C) replacing isoleucine at 359 position with leucine was then introduced by ordinary methods. Two types of enzyme preparations, human cytochrome P450 CYP2C9 (359I1e) and (359Leu). were prepared by methods described in the literature and used in the following experiment.

### (Measurement of human cytochrome P450 CYP2C9)

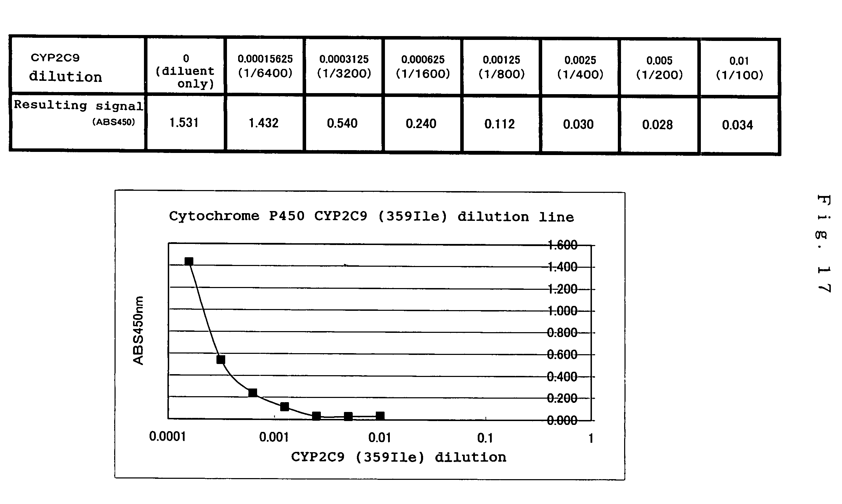

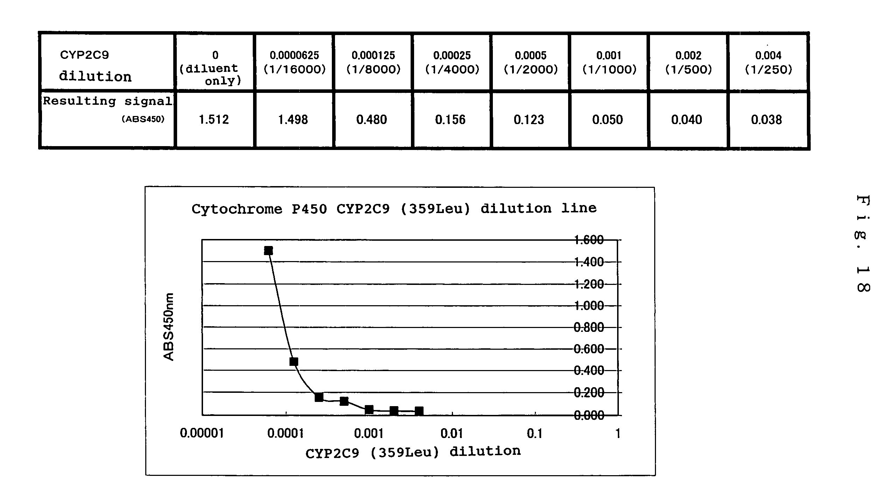

The two human cytochrome P450 CYP2C9 preparations were diluted with 100 mM phosphoric acid buffer (pH 7.2, containing 0.1% BSA: phosphoric acid buffer 2), and concentrations sequences prepared. 25 µL of each concentration together with 100 µL of a reaction mixture with phosphoric acid buffer 2 as the base containing 0.5 units/ml G6PDH, 4.13 mM MgCl₂, 1.63 mM NADP 2Na, 4.13 mM G6P Na and 62.5 µM dibenzylfluorescein (from BD Science) was mixed in 96-well polypropylene assay plates together with 25 µL of phosphoric acid buffer 2, and reacted for 20 minutes at 37°C. 50 µL of this reaction liquid was taken and mixed together with 50 µL of peroxidase-labeled fluorescein (E2-HRP) from Sigma Co. having 10 mM phosphoric acid buffer as the base (pH 7.2, containing 0.1% BSA: phosphoric acid buffer 3) on anti-fluorescein antibody-coated plates from Cosmo Bio Co., and reacted for 1 hour at 4°C. Following the reaction the plates were washed three times with 200 µL of phosphoric acid buffer 1 containing 0.1% Tween 20, and 100 µL of an enzyme reaction solution containing tetramethylbenzidine was added and incubated for 20 minutes at 37° C. Next the enzyme reaction was halted with 100 µL of 1N sulfuric acid, and absorbance was measured at 450 nm using a microplate reader, and the data processed. The relationship between human cytochrome P450 CYP2C9 concentration and the resulting absorbance is shown in Figure 17 and 18. There is an extremely high correlation between between human cytochrome P450 CYP2C9 concentration and absorbance, indicating that using the method of this example it is possible to measure human cytochrome P450 CYP2C9 activity in a sample from the absorbance data.

### [Example 4B]

### (Evaluation of the inhibitory effect of a chemical substance on human cytochrome P450 CYP2C9)

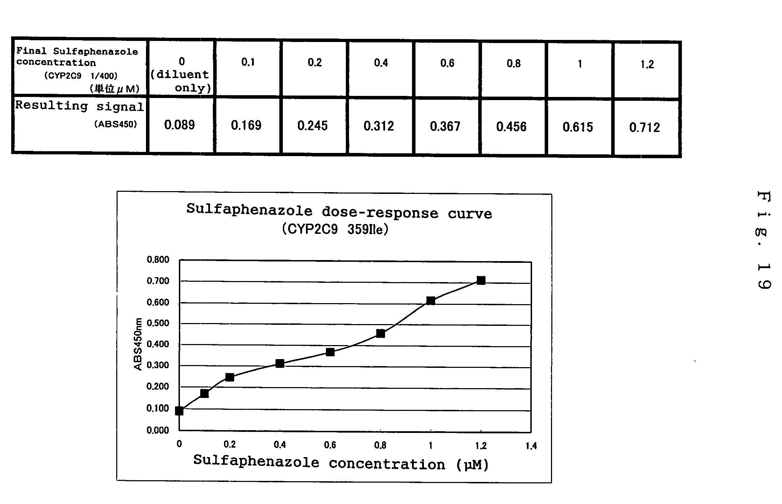

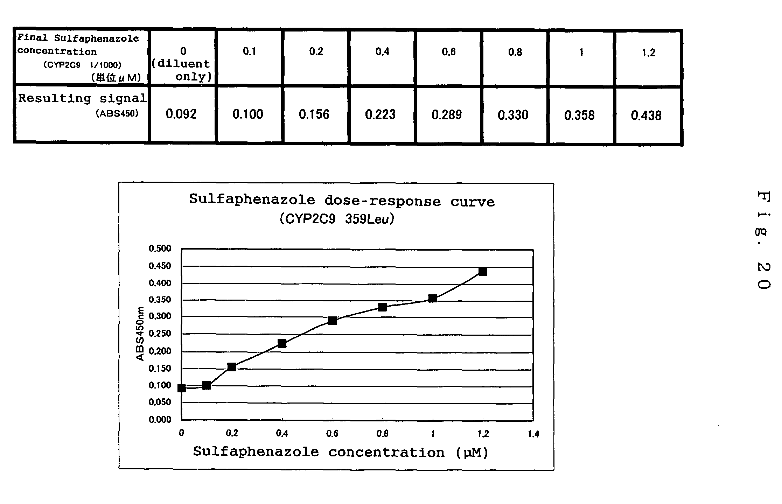

The two human cytochrome P450 CYP2C9 preparations, (359I1e) and (359Leu), were diluted about 400 times and 1000 times with phosphoric acid buffer 2, and 25 µL of each cytochrome P450 CYP2C9 solution and 100 µL of a reaction mixture with phosphoric acid buffer 2 as the base containing 0.5 units/ml G6PDH, 4.13 mM MgCl₂, 1.63 mM NADP 2Na, 4.13 mM G6P Na and 30 µM dibenzylfluorescein together with 25 µL of a 0-1.2 µM (final concentration) solution of BD Science, sulfaphenazole were mixed on 96-well polypropylene assay plates and reacted for 20 minutes at 37°C. 50 µL of this reaction liquid was taken and subjected to the same operations as in Example 1B, and the relationship between absorbance at 450 nm and sulfaphenazole solution concentration was examined. The correspondences are shown in Figures 19 and 20. The greater the amount of sulfaphenazole, the greater the inhibition of the two types of human cytochrome P450 CYP2C9, with less fluorescein produced and the signal strength increasing in the enzyme immunoassay method. There are obvious differences between the sulfaphenazole inhibition-dose curves of the two different enzyme preparations.

Using the present invention, aromatase activity can be measured easily, rapidly and efficiently without the use of radioactive compounds. It is also possible with this invention to easily, rapidly and efficiently evaluate the inhibitory actions of several chemical substances on drug-metabolizing enzymes without using radioactive chemical substances. Because of this speed and ease of use, this invention is particularly suitable for evaluating disrupting chemical substances and drugs which target drug-metabolizing enzymes.

With the present invention, it is also possible to easily, speedily and efficiently measure drug-metabolizing enzymes without using radioactive compounds. It is also possible with this invention to easily, rapidly and efficiently evaluate the inhibitory actions of several chemical substances on wild-type and mutated drug-metabolizing enzymes without using radioactive compounds. Because of this speed and ease of use, this invention is particularly suitable for evaluating disrupting chemical substances and drugs which target wild-type and mutated drug-metabolizing enzymes.

## Claims

1. A method of evaluating the degree to which a certain substance or substances are affected by other substance or substances which may have an effect on intended action of said certain substance or substances wherein said other substance or substances are first applied, and then the intended action of said certain substance or substances is determined by assaying the resulting product by an immunochemical method.

2. A method of evaluating the degree of effect of a third substance or substances on the effect that other substance or substances may have on the intended action of a certain substance or substances, comprising the steps of:
applying said other substance or substances to said certain substance or substances in the presence of the third substance or substances; and
measuring changes in the amount of the resulting product by an immunochemical method to determine the intended action of said certain substance or substances.

3. A method of measuring a drug-metabolizing enzyme activity the method comprising the steps of:
applying the drug-metabolizing enzyme to a substrate or substrates (particularly an endogenous unmodified substrate or substrates); and
assaying the resulting product by an immunochemical method, preferably within 3 hours.

4. A method of measuring drug-metabolizing enzyme activity comprising the steps of:
applying the drug-metabolizing enzyme to a substrate or substrates (particularly an endogenous unmodified substrate or substrates) in the presence of a chemical substance or substrates to be evaluated; and
determining changes in the amount of the resulting product by an immunochemical method, with preferably at least 10 samples, more preferably at least 28 samples, or further more preferably at least 96 samples measured within 3 hours, and the effect of the chemical substance of interest is evaluated, to evaluate the effect of the chemical substance to be evaluated.

5. The method according to any one of claims 1 to 4, wherein the immunochemical method employs at least one antibody which specifically recognizes the product.

6. The method according to claim 5, wherein one or more methods from among enzyme immunoassay, surface plasmon resonance, micro-differential thermal measurement and quartz resonance are employed using at least one antibody which specifically recognizes the product.

7. The method according to claim 3 or 4, wherein the drug-metabolizing enzyme is one or a mixture of two or more enzymes selected from the group consisting of proteins corresponding to genes CYP1A-B, CYP2A-2M, CYP3A1, CYP4A-4S, CYP5, CYP6A-6W, CYP7A-B, CYP8, CYP8B, CYP9B, CYP9C, CYP9F, CYP9H, CYP10, CYP11, CYP11A-B, CYP12-12E, CYP13A, CYP17, CYP19, CYP19A, CYP18A, CYP21, CYP21A, CYP24, CYP26, CYP26A, CYP27A-27BB, CYP28A-28D, CYP36A, CYP39, CYP44, CYP46, CYP49A, CYP51, CYP52A, CYP53A, CYP55A, CYP56. CYP57A, CYP58, CYP59A, CYP60A-60B, CYP61, CYP62, CYP64, CYP65A, CYP67, CYP71A, CYP71B, CYP71C, CYP71D, CYP71E, CYP73A, CYP74A CYP75A, CYP76A-76C, CYP77A, CYP78A, CYP79A-79B, CYP80A-80E, CYP82A, CYP83A-83B, CYP84A, CYP85, CYP86A, CYP88A, CYP89A, CYP90A-90C, CYP91A, CYP93A-93B, CYP94A, CYP97B, CYP98A, CYP99A, CYP101, CYP102A, CYP103, CYP104, CYP105A-105E, CYP106, CYP106A, CYP107A-107J, CYP108, CYP109, CYP110, CYP112, CYP112A, CYP113A, CYP114, CYP116-117, CYP117A, CYP119-126, CYP127A, CYP128-132, CYP133B, CYP134, CYP135A, CYP135B, CYP136-144, CYP152A, CYP301A, CYP302A, CYP303A, CYP304A, CYP305A, CYP306A, CYP307A, CYP308A, CYP309A, CYP310A, CYP311A, CYP312A, CYP313A-313B, CYP314A, CYP315A, CYP316A, CYP317A, CYP318A and their subfamilies and subgroups, and hydroxysteroid dehydrogenase.

8. The method according to claim 3 or 4, wherein the drug-metabolizing enzyme is one or a mixture of two or more enzymes selected from the group of proteins corresponding to genes CYP1A1, CYP1A2, CYP1B1, CYP2A6, CYP2B6, CYP2C8, CYP2C91, CYP2C92, CYP2C93, CYP2C18, CYP2C19, CYP2D61, CYP2D610, CYP2E1, CYP3A4, CYP3A5, CYP3A7, CYP4A11, CYP4F2, CYP4F3A and CYP19, and 17β-hydroxysteroid dehydrogenase.

9. The method according to claim 3 or 4, wherein the drug-metabolizing enzyme is aromatase (CYP19).

10. The method according to claim 3 or 4, wherein the substrate or substrates used in measuring drug-metabolizing enzyme activity are an endogenous hormone compound or compounds.

11. The method according to claim 3 or 4, wherein the substrate or substrates used in measuring drug-metabolizing enzyme activity are classified as an androgen.

12. The method according to claim 3 or 4, wherein the substrate or substrates used in measuring drug-metabolizing enzyme activity are at least one selected from the group consisting of testosterone, progesterone, dihydrotestosterone, androstendione, 7-ethoxy-3-cyanocoumarin, 7-methoxy-4-trifluoromethylcoumarin, 7-benzyloxy-4-trifluoromethylcoumarin, dibenzylfluorescein, 7-ethoxy-4-trifluoromethylcoumarin, caffeine, acetaminophen, 7-benzyloxyquinoline, bufuralol, dapsone, debrisquin sulfate, dextromethorphan hydrobromide, diclofenac, haloperidol, 6β-hydroxycortisol, 11α-hydroxytestosterone, mephenytoin, methoxyresorufin, 3-0-methylfluorescein, midazolam, omeprazole, taxol, tienil-3C-alcohol, tolbutamide, tramadol, R-(+)-warfarin and S-(-)-warfarin.

13. The method according to claim 3 or 4, wherein the drug-metabolizing enzyme is immobilized either during or after the drug-metabolizing enzyme reaction.

14. The method according to claim 3 or 4, wherein the drug-metabolizing enzyme and other reaction components are separated after completion of the drug-metabolizing enzyme reaction.

15. The method according to claim 3 or 4, wherein heat treatment is applied or an inhibitor added to suppress the activity of the drug-metabolizing enzyme after the drug-metabolizing enzyme reaction.

16. The method according to claim 5, wherein the antibodies are polyclonal antibodies derived from fish, amphibians, birds, reptiles or mammals, or else monoclonal antibodies.

17. The method according to claim 3 or 4, wherein the drug-metabolizing enzyme is derived from shellfish, mollusks, fish, amphibians, birds, reptiles or mammals.

18. The method according to claim 3 or 4, wherein the drug-metabolizing enzyme is produced by gene recombination.

19. The method according to claim 3 or 4, wherein the antibodies are immobilized.

20. The method according to claim 3 or 4, wherein a recovery system for supplying the NADPH required by the drug-metabolizing enzyme reaction is included during the drug-metabolizing enzyme reaction.

21. The method according to claim 3 or 4, wherein the recovery system for supplying the NADPH required by the drug-metabolizing enzyme reaction contains glucose-6-phosphoric acid dehydrogenase or isocitric acid dehydrogenase.

22. The method according to claim 3 or 4, wherein the substrate of the drug-metabolizing enzyme reaction is adjusted to a concentration at which it has practically no effect on the immune reaction of the antibodies.

23. A reagent composition for measuring the degree to which a certain substance or substances are affected by other substance or substances which may have an effect on an intended action of said certain substance or substances, the reagent composition comprising (1)-(3):
(1) other substance or substances which may have an effect on the intended action of said certain substance or substances,
(2) at least one antibody which specifically recognizes the product that results when a certain substance or substances are affected by other substance or substances which may have an effect on an intended action of said certain substance or substances,
(3) at least one reagent which allows measurement using the antibody described in (2) according to one of the methods consisting of enzyme immunoassay, surface plasmon resonance, micro-differential thermal measurement or quartz resonance.

24. A kit for measuring the degree to which a certain substance or substances are affected by other substance or substances which may have an effect on an intended action of said certain substance or substances, the component parts of which are selected as needed from among at least one plate immobilizing a specific antibody or antibodies (a protein or proteins to cross-link a product or products) or at least one gold thin-film chip immobilizing a specific antibody or antibodies (a protein or proteins to cross-link a product or products), at least one quartz resonator chip immobilizing a specific antibody or antibodies (a protein or proteins to cross-link a product or products), at least one reaction buffer, at least one specific antibody solution, at least one antibody sample diluent, at least one standard substance, at least one tracer enzyme substrate liquid for assaying the tracer enzyme, and the like.

25. The kit according to claim 24, wherein the other substance is aromatase.

26. The method according to claim 3 or 4, wherein non-steroid chemical substances are evaluated.

27. The method according to claim 3 or 4, wherein the substrate concentration for the drug-metabolizing enzyme reaction is in the range of 0.05 nM to 500 µM.

28. A method of evaluating differences between individuals or between groups of humans in metabolizing enzyme activity with respect to a drug, comprising the steps of:
applying a drug-metabolizing enzyme to the drug; and
assaying the resulting product by an immunochemical method to determine an activity of the drug-metabolizing enzyme.

29. A method of evaluating differences between individuals or between groups of humans in inhibition of drug-metabolizing enzyme activity with respect to a drug, comprising the steps of:
applying the drug-metabolizing enzyme to a substrate or substrates in the presence of a chemical substance or substances to be evaluated; and
measuring changes in the amount of the resulting product by an immunochemical method to assay the activity of the drug-metabolizing enzyme.

30. The measurement method of claim 28 or 29, wherein a drug-metabolizing enzyme present in an individual or shared by a specific group of humans is a single-nucleotide polymorphism (one occurring in 1% or more of the total population) of the basic nucleotide sequence of the drug-metabolizing enzyme.

31. The measurement method of claim 28 or 29 wherein a drug-metabolizing enzyme present in an individual or shared by a specific group of humans has a mutation in its basic nucleotide sequence of the drug-metabolizing enzyme.

32. A reagent composition for evaluating differences between individuals or between specific groups of humans in metabolizing enzyme activity with respect to a drug, the composition comprising (1)-(3) below:
(1) at least one drug-metabolizing enzyme;
(2) at least one antibody which specifically recognizes the product formed by the action of the drug-metabolizing enzyme from a certain substance or substances; and
(3) at least one reagent which allows measurement of the product using the antibody described in (2), using one of the methods consisting of enzyme immunoassay, surface plasmon resonance, micro-differential thermal measurement and quartz resonance.

33. A reagent composition for evaluating differences between individuals or between specific groups of humans in the inhibition of the activity of a metabolizing enzyme with respect to a drug, the composition comprising (1)-(3) below:
(1) at least one drug-metabolizing enzyme;
(2) at least one antibody which specifically recognizes the product which results when a substance is acted upon by the drug-metabolizing enzyme; and
(3) at least one reagent which allows measurement using the antibody described in (2), using one of the methods consisting of enzyme immunoassay, surface plasmon resonance, micro-differential thermal measurement, and quartz resonance.

34. A kit for evaluating differences between individuals or between specific groups of humans in metabolizing enzyme activity with respect to a drug, the component parts of which are selected as needed from among at least one plate immobilizing a specific antibody or antibodies (a protein or proteins to cross-link a product or products) or at least one gold thin-film chip immobilizing a specific antibody or antibodies (a protein or proteins to cross-link a product or products), at least one quartz resonator chip immobilizing a specific antibody or antibodies (a protein or proteins to cross-link a product or products), at least one reaction buffer, at least one specific antibody solution, at least one antibody sample diluent, at least one standard substance, at least one tracer enzyme substrate liquid for assaying the tracer enzyme, and the like.

35. A kit for evaluating differences between individuals or between specific groups of humans in inhibition of metabolizing enzyme activity with respect to a drug, the component parts of which are selected as needed from among at least one plate immobilizing a specific antibody or antibodies (a protein or proteins to cross-link a product or products) or at least one gold thin-film chip immobilizing a specific antibody or antibodies (a protein or proteins to cross-link a product or products), at least one quartz resonator chip immobilizing a specific antibody or antibodies (a protein or proteins to cross-link a product or products), at least one reaction buffer, at least one specific antibody solution, at least one antibody sample diluent, at least one standard substance, at least one tracer enzyme substrate liquid for assaying the tracer enzyme, and the like.
